# EUROPEAN PATENT APPLICATION

(11) **EP 2 006 686 A1**
(43) Date of publication of application: **24.12.2008**
(21) Application number: 07290782.7
(22) Date of filing: 22.06.2007
(51) Int. Cl.: G01N 33/68

(54) **Method for screening for selective modulator of the NF-kB pathway activation**

(71) Applicant: INSTITUT PASTEUR, 75015 Paris (FR); Centre National de la Recherche Scientifique, 75016 Paris (FR)
(72) Inventor: Agou, Fabrice, 75012 Paris (FR); Chiaravalli, Jeanne, 75015 Paris (FR); Coïc, Yves Marie, 92190 Meudon (FR); Baleux, Françoise, 75005 Paris (FR); Israel, Alain, 75014 Paris (FR); Veron, Michel, 75004 Paris (FR)
(74) Representative: Vialle-Presles, Marie José

(57) **Abstract**

The invention relates to methods for screening for selective modulator of NF-κB pathway activation. The invention concerns methods for primary screening molecules that potentially modulate (activate or inhibit) the NF-kB pathway activation by identifying and selecting molecules modulate the interaction of NEMO with other proteins. The invention also concerns methods for secondary screening for modulator (activator or inhibitor) of the NF-kB pathway activation.

SEQUENCE LISTING

<110> INSTITUT PASTEUR
Centre National de la Recherche Scientifique

<120> METHOD FOR SCREENING FOR SELECTIVE MODULATOR OF THE NF-kB PATHWAY
ACTIVATION

<130> BLO/TC/cp-F226Cas138EP

<160> 27

<170> PatentIn version 3.3

<210> 1
<211> 412
<212> PRT
<213> Mus musculus

<400> 1

Met Asn Lys His Pro Trp Lys Asn Gln Leu Ser Glu Thr Val Gln Pro
1               5                   10                  15

Ser Gly Gly Pro Ala Glu Asp Gln Asp Met Leu Gly Glu Glu Ser Ser
            20                  25                  30

Leu Gly Lys Pro Ala Met Leu His Leu Pro Ser Glu Gln Gly Thr Pro
        35                  40                  45

Glu Thr Leu Gln Arg Cys Leu Glu Glu Asn Gln Glu Leu Arg Asp Ala
    50                  55                  60

Ile Arg Gln Ser Asn Gln Met Leu Arg Glu Arg Cys Glu Glu Leu Leu
65                  70                  75                  80

His Phe Gln Val Ser Gln Arg Glu Glu Lys Glu Phe Leu Met Cys Lys
                85                  90                  95

Phe Gln Glu Ala Arg Lys Leu Val Glu Arg Leu Ser Leu Glu Lys Leu
            100                 105                 110

Asp Leu Arg Ser Gln Arg Glu Gln Ala Leu Lys Glu Leu Glu Gln Leu
        115                 120                 125

Lys Lys Cys Gln Gln Gln Met Ala Glu Asp Lys Ala Ser Val Lys Ala
    130                 135                 140

Gln Val Thr Ser Leu Leu Gly Glu Leu Gln Glu Ser Gln Ser Arg Leu
145                 150                 155                 160

Glu Ala Ala Thr Lys Asp Arg Gln Ala Leu Glu Gly Arg Ile Arg Ala
                165                 170                 175

Val Ser Glu Gln Val Arg Gln Leu Glu Ser Glu Arg Glu Val Leu Gln
            180                 185                 190

Gln Gln His Ser Val Gln Val Asp Gln Leu Arg Met Gln Asn Gln Ser
        195                 200                 205

Val Glu Ala Ala Leu Arg Met Glu Arg Gln Ala Ala Ser Glu Glu Lys
    210                 215                 220

Arg Lys Leu Ala Gln Leu Gln Ala Ala Tyr His Gln Leu Phe Gln Asp
225                 230                 235                 240

Tyr Asp Ser His Ile Lys Ser Ser Lys Gly Met Gln Leu Glu Asp Leu
                245                 250                 255

Arg Gln Gln Leu Gln Gln Ala Glu Glu Ala Leu Val Ala Lys Gln Glu
            260                 265                 270

Leu Ile Asp Lys Leu Lys Glu Glu Ala Glu Gln His Lys Ile Val Met
        275                 280                 285

Glu Thr Val Pro Val Leu Lys Ala Gln Ala Asp Ile Tyr Lys Ala Asp
    290                 295                 300

Phe Gln Ala Glu Arg His Ala Arg Glu Lys Leu Val Glu Lys Lys Glu
305                 310                 315                 320

Tyr Leu Gln Glu Gln Leu Glu Gln Leu Gln Arg Glu Phe Asn Lys Leu
                325                 330                 335

Lys Val Gly Cys His Glu Ser Ala Arg Ile Glu Asp Met Arg Lys Arg
            340                 345                 350

His Val Glu Thr Pro Gln Pro Pro Leu Leu Pro Ala Pro Ala His His
        355                 360                 365

Ser Phe His Leu Ala Leu Ser Asn Gln Arg Arg Ser Pro Pro Glu Glu
    370                 375                 380

Pro Pro Asp Phe Cys Cys Pro Lys Cys Gln Tyr Gln Ala Pro Asp Met
385                 390                 395                 400

Asp Thr Leu Gln Ile His Val Met Glu Cys Ile Glu
                405                 410

<210> 2
<211> 419
<212> PRT
<213> Homo sapiens

<400> 2

Met Asn Arg His Leu Trp Lys Ser Gln Leu Cys Glu Met Val Gln Pro
1               5                   10                  15

Ser Gly Gly Pro Ala Ala Asp Gln Asp Val Leu Gly Glu Glu Ser Pro
            20                  25                  30

Leu Gly Lys Pro Ala Met Leu His Leu Pro Ser Glu Gln Gly Ala Pro
        35                  40                  45

Glu Thr Leu Gln Arg Cys Leu Glu Glu Asn Gln Glu Leu Arg Asp Ala
    50                  55                  60

Ile Arg Gln Ser Asn Gln Ile Leu Arg Glu Arg Cys Glu Glu Leu Leu
65                  70                  75                  80

His Phe Gln Ala Ser Gln Arg Glu Glu Lys Glu Phe Leu Met Cys Lys
                85                  90                  95

Phe Gln Glu Ala Arg Lys Leu Val Glu Arg Leu Gly Leu Glu Lys Leu
            100                 105                 110

Asp Leu Lys Arg Gln Lys Glu Gln Ala Leu Arg Glu Val Glu His Leu
        115                 120                 125

Lys Arg Cys Gln Gln Gln Met Ala Glu Asp Lys Ala Ser Val Lys Ala
    130                 135                 140

Gln Val Thr Ser Leu Leu Gly Glu Leu Gln Glu Ser Gln Ser Arg Leu
145                 150                 155                 160

Glu Ala Ala Thr Lys Glu Cys Gln Ala Leu Glu Gly Arg Ala Arg Ala
                165                 170                 175

Ala Ser Glu Gln Ala Arg Gln Leu Glu Ser Glu Arg Glu Ala Leu Gln
            180                 185                 190

Gln Gln His Ser Val Gln Val Asp Gln Leu Arg Met Gln Gly Gln Ser
        195                 200                 205

Val Glu Ala Ala Leu Arg Met Glu Arg Gln Ala Ala Ser Glu Glu Lys
    210                 215                 220

Arg Lys Leu Ala Gln Leu Gln Val Ala Tyr His Gln Leu Phe Gln Glu
225                 230                 235                 240

Tyr Asp Asn His Ile Lys Ser Ser Val Val Gly Ser Glu Arg Lys Arg
                245                 250                 255

Gly Met Gln Leu Glu Asp Leu Lys Gln Gln Leu Gln Gln Ala Glu Glu
            260                 265                 270

Ala Leu Val Ala Lys Gln Glu Val Ile Asp Lys Leu Lys Glu Glu Ala
        275                 280                 285

Glu Gln His Lys Ile Val Met Glu Thr Val Pro Val Leu Lys Ala Gln
    290                 295                 300

Ala Asp Ile Tyr Lys Ala Asp Phe Gln Ala Glu Arg Gln Ala Arg Glu
305                 310                 315                 320

Lys Leu Ala Glu Lys Lys Glu Leu Leu Gln Glu Gln Leu Glu Gln Leu
                325                 330                 335

Gln Arg Glu Tyr Ser Lys Leu Lys Ala Ser Cys Gln Glu Ser Ala Arg
            340                 345                 350

Ile Glu Asp Met Arg Lys Arg His Val Glu Val Ser Gln Ala Pro Leu
        355                 360                 365

Pro Pro Ala Pro Ala Tyr Leu Ser Ser Pro Leu Ala Leu Pro Ser Gln
    370                 375                 380

Arg Arg Ser Pro Pro Glu Glu Pro Pro Asp Phe Cys Cys Pro Lys Cys
385                 390                 395                 400

Gln Tyr Gln Ala Pro Asp Met Asp Thr Leu Gln Ile His Val Met Glu
                405                 410                 415

Cys Ile Glu

<210>  3
<211>  17
<212>  PRT
<213>  Artificial Sequence

<220>
<223>  BA

<400>  3

Cys Arg Gln Ile Lys Ile Trp Phe Gln Asn Arg Arg Met Lys Trp Lys
1               5                   10                  15

Lys

<210>  4
<211>  60
<212>  PRT
<213>  Artificial Sequence

<220>
<223>  m BA-LZ

<400>  4

Cys Arg Gln Ile Lys Ile Trp Phe Gln Asn Arg Arg Met Lys Trp Lys
1               5                   10                  15

Lys Leu Lys Ala Gln Ala Asp Ile Tyr Lys Ala Asp Phe Gln Ala Glu
            20                  25                  30

Arg His Ala Arg Glu Lys Leu Val Glu Lys Lys Glu Tyr Leu Gln Glu
        35                  40                  45

Gln Leu Glu Gln Leu Gln Arg Glu Phe Asn Lys Leu
    50                  55                  60

<210>  5
<211>  60
<212>  PRT
<213>  Artificial Sequence

<220>
<223>  h BA-LZ

<400>  5

Cys Arg Gln Ile Lys Ile Trp Phe Gln Asn Arg Arg Met Lys Trp Lys
1               5                   10                  15

Lys Leu Lys Ala Gln Ala Asp Ile Tyr Lys Ala Asp Phe Gln Ala Glu
            20                  25                  30

Arg Gln Ala Arg Glu Lys Leu Ala Glu Lys Lys Glu Leu Leu Gln Glu
        35                  40                  45

Gln Leu Glu Gln Leu Gln Arg Glu Tyr Ser Lys Leu
    50                  55                  60

<210>  6
<211>  43
<212>  PRT
<213>  Artificial Sequence

<220>
<223>  m LZ

<400>  6

Leu Lys Ala Gln Ala Asp Ile Tyr Lys Ala Asp Phe Gln Ala Glu Arg
1               5                   10                  15

His Ala Arg Glu Lys Leu Val Glu Lys Lys Glu Tyr Leu Gln Glu Gln
            20                  25                  30

Leu Glu Gln Leu Gln Arg Glu Phe Asn Lys Leu
        35                  40

<210>  7
<211>  43
<212>  PRT
<213>  Artificial Sequence

<220>
<223>  h LZ

<400>  7

Leu Lys Ala Gln Ala Asp Ile Tyr Lys Ala Asp Phe Gln Ala Glu Arg
1               5                   10                  15

Gln Ala Arg Glu Lys Leu Ala Glu Lys Lys Glu Leu Leu Gln Glu Gln
            20                  25                  30

Leu Glu Gln Leu Gln Arg Glu Tyr Ser Lys Leu
        35                  40

<210>  8
<211>  40
<212>  PRT
<213>  Artificial

<220>
<223>  m CC2

<400>  8

Ser Lys Gly Met Gln Leu Glu Asp Leu Arg Gln Gln Leu Gln Gln Ala
1               5                   10                  15

Glu Glu Ala Leu Val Ala Lys Gln Glu Leu Ile Asp Lys Leu Lys Glu
            20                  25                  30

Glu Ala Glu Gln His Lys Ile Val
        35                  40

<210>  9
<211>  40
<212>  PRT
<213>  Artificial

<220>
<223>  h CC2

<400>  9

Ser Lys Gly Met Gln Leu Glu Asp Leu Lys Gln Gln Leu Gln Gln Ala
1               5                   10                  15

Glu Glu Ala Leu Val Ala Lys Gln Glu Val Ile Asp Lys Leu Lys Glu
            20                  25                  30

Glu Ala Glu Gln His Lys Ile Val
        35                  40

<210>  10
<211>  21
<212>  PRT
<213>  Artificial

<220>
<223>  m NLM

<400>  10

Leu Lys Ala Gln Ala Asp Ile Tyr Lys Ala Asp Phe Gln Ala Glu Arg
1               5                   10                  15

His Ala Arg Glu Lys
            20

<210>  11
<211>  21
<212>  PRT
<213>  Artificial Sequence

<220>
<223>  m DR NLM

<400>  11

Leu Lys Ala Gln Ala Asp Ile Tyr Lys Ala Arg Phe Gln Ala Glu Arg
1               5                   10                  15

His Ala Arg Glu Lys
            20

<210>  12
<211>  21
<212>  PRT
<213>  Artificial

<220>
<223>  h DR NLM

<400>  12

Leu Lys Ala Gln Ala Asp Ile Tyr Lys Ala Arg Phe Gln Ala Glu Arg
1               5                   10                  15

Gln Ala Arg Glu Lys
            20

<210>  13
<211>  38
<212>  PRT
<213>  Artificial Sequence

<220>
<223>  m BA-DR NLM

<400>  13

Cys Arg Gln Ile Lys Ile Trp Phe Gln Asn Arg Arg Met Lys Trp Lys
1               5                   10                  15

Lys Leu Lys Ala Gln Ala Asp Ile Tyr Lys Ala Arg Phe Gln Ala Glu
            20                  25                  30

Arg His Ala Arg Glu Lys
        35

<210>  14
<211>  38

<212>  PRT
<213>  Artificial

<220>
<223>  h BA-DR NLM

<400>  14

Cys Arg Gln Ile Lys Ile Trp Phe Gln Asn Arg Arg Met Lys Trp Lys
1               5                   10                  15

Lys Leu Lys Ala Gln Ala Asp Ile Tyr Lys Ala Arg Phe Gln Ala Glu
            20                  25                  30

Arg Gln Ala Arg Glu Lys
        35

<210>  15
<211>  29
<212>  PRT
<213>  Artificial Sequence

<220>
<223>  m BR7-DR NLM

<400>  15

Cys Arg Arg Arg Arg Arg Arg Arg Leu Lys Ala Gln Ala Asp Ile Tyr
1               5                   10                  15

Lys Ala Arg Phe Gln Ala Glu Arg His Ala Arg Glu Lys
            20                  25

<210>  16
<211>  29
<212>  PRT
<213>  Artificial

<220>
<223>  h BR7-DR NLM

<400>  16

Cys Arg Arg Arg Arg Arg Arg Arg Leu Lys Ala Gln Ala Asp Ile Tyr
1               5                   10                  15

Lys Ala Arg Phe Gln Ala Glu Arg Gln Ala Arg Glu Lys
            20                  25

<210>  17
<211>  84
<212>  PRT
<213>  Artificial

<220>
<223>  CC2-LZ

<400>  17

Leu Glu Asp Leu Arg Gln Gln Leu Gln Gln Ala Glu Glu Ala Leu Val
1               5                   10                  15

Ala Lys Gln Glu Leu Ile Asp Lys Leu Lys Glu Glu Ala Glu Gln His
            20                  25                  30

Lys Ile Val Met Glu Thr Val Pro Val Leu Lys Ala Gln Ala Asp Ile
        35                  40                  45

Tyr Lys Ala Asp Phe Gln Ala Glu Arg His Ala Arg Glu Lys Leu Val
    50                  55                  60

Glu Lys Lys Glu Tyr Leu Gln Glu Gln Leu Glu Gln Leu Gln Arg Glu
65                  70                  75                  80

Phe Asn Lys Leu

<210>  18
<211>  21
<212>  PRT
<213>  Artificial

<220>
<223>  h NLM

<400>  18

Leu Lys Ala Gln Ala Asp Ile Tyr Lys Ala Asp Phe Gln Ala Glu Arg
1               5                   10                  15

His Ala Arg Glu Lys
            20

<210>  19
<211>  84
<212>  PRT
<213>  Artificial

<220>
<223>  h CC2-LZ

<400>  19

Leu Glu Asp Leu Lys Gln Gln Leu Gln Gln Ala Glu Glu Ala Leu Val
1               5                   10                  15

Ala Lys Gln Glu Val Ile Asp Lys Leu Lys Glu Glu Ala Glu Gln His
            20                  25                  30

Lys Ile Val Met Glu Thr Val Pro Val Leu Lys Ala Gln Ala Asp Ile
        35                  40                  45

Tyr Lys Ala Asp Phe Gln Ala Glu Arg Gln Ala Arg Glu Lys Leu Ala
    50                  55                  60

Glu Lys Lys Glu Leu Leu Gln Glu Gln Leu Glu Gln Leu Gln Arg Glu
65                  70                  75                  80

Tyr Ser Lys Leu

<210> 20
<211> 38
<212> PRT
<213> Artificial

<220>
<223> murine A-Cterm LZ

<400> 20

Arg Gln Ile Lys Ile Trp Phe Gln Asn Arg Arg Met Lys Trp Lys Lys
1               5                   10                  15

Leu Val Glu Lys Lys Glu Tyr Leu Gln Glu Gln Leu Glu Gln Leu Gln
            20                  25                  30

Arg Glu Phe Asn Lys Leu
        35

<210> 21
<211> 38
<212> PRT
<213> Artificial

<220>
<223> h A-Cterm-LZ

<400> 21

Arg Gln Ile Lys Ile Trp Phe Gln Asn Arg Arg Met Lys Trp Lys Lys
1               5                   10                  15

Leu Ala Glu Lys Lys Glu Leu Leu Gln Glu Gln Leu Glu Gln Leu Gln
            20                  25                  30

Arg Glu Tyr Ser Lys Leu
        35

<210> 22
<211> 60
<212> PRT
<213> Artificial

<220>
<223> BA-LZ L322P/L329P

<400> 22

Cys Arg Gln Ile Lys Ile Trp Phe Gln Asn Arg Arg Met Lys Trp Lys
1               5                   10                  15

Lys Leu Lys Ala Gln Ala Asp Ile Tyr Lys Ala Asp Phe Gln Ala Glu
            20                  25                  30

Arg His Ala Arg Glu Lys Leu Val Glu Lys Lys Glu Tyr Pro Gln Glu
        35                  40                  45

Gln Leu Glu Gln Pro Gln Arg Glu Phe Asn Lys Leu
    50                  55                  60

<210>  23
<211>  8
<212>  PRT
<213>  Artificial

<220>
<223>  Flag

<400>  23

Asp Tyr Lys Asp Asp Asp Asp Lys
1               5

<210>  24
<211>  2787
<212>  DNA
<213>  Artificial

<220>
<223>  pcDNA3-Flag-NEMO

<220>
<221>  CDS
<222>  (911)..(2209)

<400>  24
gacggatcgg gagatctccc gatcccctat ggtcgactct cagtacaatc tgctctgatg      60

ccgcatagtt aagccagtat ctgctccctg cttgtgtgtt ggaggtcgct gagtagtgcg     120

cgagcaaaat ttaagctaca acaaggcaag gcttgaccga caattgcatg aagaatctgc     180

ttagggttag gcgttttgcg ctgcttcgcg atgtacgggc cagatatacg cgttgacatt     240

gattattgac tagttattaa tagtaatcaa ttacggggtc attagttcat agcccatata     300

tggagttccg cgttacataa cttacggtaa atggcccgcc tggctgaccg cccaacgacc     360

cccgcccatt gacgtcaata atgacgtatg ttcccatagt aacgccaata gggactttcc     420

attgacgtca atgggtggac tatttacggt aaactgccca cttggcagta catcaagtgt     480

atcatatgcc aagtacgccc cctattgacg tcaatgacgg taaatggccc gcctggcatt     540

atgcccagta catgacctta tgggactttc ctacttggca gtacatctac gtattagtca     600

tcgctattac catggtgatg cggttttggc agtacatcaa tgggcgtgga tagcggtttg     660

actcacgggg atttccaagt ctccacccca ttgacgtcaa tgggagtttg ttttggcacc     720

aaaatcaacg ggactttcca aaatgtcgta acaactccgc cccattgacg caaatgggcg     780

gtaggcgtgt acggtgggag gtctatataa gcagagctct ctggctaact agagaaccca     840

ctgcttactg gcttatcgaa attaatacga ctcactatag ggagacccta gaactagtgg     900

atccgccacc atg gac tac aag gac gac gat gac aaa gat ccc ccg atc        949
           Met Asp Tyr Lys Asp Asp Asp Asp Lys Asp Pro Pro Ile

                    1               5                   10

tct cga gga tcc gaa ttc ctc gac aac aag cac ccc tgg aag aac cag        997
Ser Arg Gly Ser Glu Phe Leu Asp Asn Lys His Pro Trp Lys Asn Gln
    15                  20                  25

ctg agt gag atg gtg cag ccc agt ggt ggc cca gca gag gac cag gac       1045
Leu Ser Glu Met Val Gln Pro Ser Gly Gly Pro Ala Glu Asp Gln Asp
30                  35                  40                  45

atg ctg ggt gaa gaa tct tct ctg ggg aag cct gca atg cta cat ctg       1093
Met Leu Gly Glu Glu Ser Ser Leu Gly Lys Pro Ala Met Leu His Leu
                50                  55                  60

cct tca gag cag ggt act cct gag acc ctc cag cgc tgc ctg gaa gag       1141
Pro Ser Glu Gln Gly Thr Pro Glu Thr Leu Gln Arg Cys Leu Glu Glu
            65                  70                  75

aat caa gag ctc cga gac gct atc cgg cag agc aat cag atg ctg agg       1189
Asn Gln Glu Leu Arg Asp Ala Ile Arg Gln Ser Asn Gln Met Leu Arg
        80                  85                  90

gaa cgc tgt gag gag ctg ctg cat ttc cag gtc agc cag cgg gag gag       1237
Glu Arg Cys Glu Glu Leu Leu His Phe Gln Val Ser Gln Arg Glu Glu
    95                  100                 105

aag gag ttc ctt atg tgc aaa ttc cag gaa gcc cgg aag ctg gtg gag       1285
Lys Glu Phe Leu Met Cys Lys Phe Gln Glu Ala Arg Lys Leu Val Glu
110                 115                 120                 125

aga ctg agc ttg gag aag ctt gat ctt cgg agt cag agg gaa cag gcc       1333
Arg Leu Ser Leu Glu Lys Leu Asp Leu Arg Ser Gln Arg Glu Gln Ala
                130                 135                 140

tta aag gag ttg gag caa ctg aag aaa tgc caa cag cag atg gct gag       1381
Leu Lys Glu Leu Glu Gln Leu Lys Lys Cys Gln Gln Gln Met Ala Glu
            145                 150                 155

gac aag gcc tct gtg aaa gtt cag gtg aca tca ttg ctc gga gaa ctc       1429
Asp Lys Ala Ser Val Lys Val Gln Val Thr Ser Leu Leu Gly Glu Leu
        160                 165                 170

cag gag agc cag agc cgt ttg gag gct gcc acc aag gat cgg caa gct       1477
Gln Glu Ser Gln Ser Arg Leu Glu Ala Ala Thr Lys Asp Arg Gln Ala
    175                 180                 185

tta gag gga agg att cga gca gtt agt gag cag gtc aga cag ctg gag       1525
Leu Glu Gly Arg Ile Arg Ala Val Ser Glu Gln Val Arg Gln Leu Glu
190                 195                 200                 205

agt gag cgg gag gtg cta cag cag cag cac agc gtc cag gtg gac cag       1573
Ser Glu Arg Glu Val Leu Gln Gln Gln His Ser Val Gln Val Asp Gln
                210                 215                 220

ctg cgt atg cag aac cag agc gtg gag gct gcc ttg cga atg ggg cgg       1621
Leu Arg Met Gln Asn Gln Ser Val Glu Ala Ala Leu Arg Met Gly Arg
            225                 230                 235

cag gct gct tca gag gag aag cgg aag ctg gct cag ttg cag gca gcc       1669
Gln Ala Ala Ser Glu Glu Lys Arg Lys Leu Ala Gln Leu Gln Ala Ala
        240                 245                 250

tat cac cag ctc ttc caa gac tac gac agc cac att aag agc aac aag       1717
Tyr His Gln Leu Phe Gln Asp Tyr Asp Ser His Ile Lys Ser Asn Lys
    255                 260                 265

ggc atg cag ctg gaa gat ctg agg caa cag ctc cag caa gct gag gag       1765
Gly Met Gln Leu Glu Asp Leu Arg Gln Gln Leu Gln Gln Ala Glu Glu

270                 275                 280                 285

gcc ctg gta gcc aaa cag gaa ttg att gat aag ctg aaa gag gag gct      1813
Ala Leu Val Ala Lys Gln Glu Leu Ile Asp Lys Leu Lys Glu Glu Ala
                290                 295                 300

gag cag cac aag att gtg atg gag act gtg cca gtc ttg aag gcc cag      1861
Glu Gln His Lys Ile Val Met Glu Thr Val Pro Val Leu Lys Ala Gln
            305                 310                 315

gcg gat atc tac aag gct gac ttc caa gct gag agg cat gcc cgg gag      1909
Ala Asp Ile Tyr Lys Ala Asp Phe Gln Ala Glu Arg His Ala Arg Glu
        320                 325                 330

aag ctg gtg gag aag aag gag tat ttg cag gag cag ctg gag cag ctg      1957
Lys Leu Val Glu Lys Lys Glu Tyr Leu Gln Glu Gln Leu Glu Gln Leu
    335                 340                 345

cag cgc gag ttc aac aag ctg aaa gtt ggc tgc cat gag tca gcc agg      2005
Gln Arg Glu Phe Asn Lys Leu Lys Val Gly Cys His Glu Ser Ala Arg
350                 355                 360                 365

att gag gat atg agg aag cgg cat gta gag act ccc cag cct act tta      2053
Ile Glu Asp Met Arg Lys Arg His Val Glu Thr Pro Gln Pro Thr Leu
                370                 375                 380

ctc cct gct cca gct cac cac tcc ttt cat ttg gcc ttg tcc aac cag      2101
Leu Pro Ala Pro Ala His His Ser Phe His Leu Ala Leu Ser Asn Gln
            385                 390                 395

cgg agg agc cct cct gaa gaa cct cct gac ttc tgt tgt ccg aag tgc      2149
Arg Arg Ser Pro Pro Glu Glu Pro Pro Asp Phe Cys Cys Pro Lys Cys
        400                 405                 410

cag tat cag gct cct gat atg gac act cta cag ata cat gtc atg gag      2197
Gln Tyr Gln Ala Pro Asp Met Asp Thr Leu Gln Ile His Val Met Glu
    415                 420                 425

tgc ata gag tag gggcagcaga tgcaaggcca cttgcagtac tatgtcctga          2249
Cys Ile Glu
430

tctgtgtgac ttgtgctttc ctgttttacc tgcatagtcc acacttaagg gcttgcttta    2309

gccctttggt cccccattta gggtagacag ccccattcag ggcttttttt tttttctgtg    2369

tgcctgatcc agtttgcctc tggtggcttc ttccctcttc tcccatagtc ctagggagtc    2429

tagagggccc tattctatag tgtcacctaa atgctagagc tcgctgatca gcctcgactg    2489

tgccttctag ttgccagcca tctgttgttt gcccctcccc cgtgccttcc ttgaccctgg    2549

aaggtgccac tcccactgtc ctttcctaat aaaatgagga aattgcatcg cattgtctga    2609

gtaggtgtca ttctattctg gggggtgggg tggggcagga cagcaagggg gaggattggg    2669

aagacaatag caggcatgct ggggatgcgg tgggctctat ggcttctgag gcggaaagaa    2729

ccagctgggg ctctaggggg tatccccacg cgccctgtag cggcgcatta agcgcggc      2787

<210>  25
<211>  432
<212>  PRT
<213>  Artificial

<220>
<223>  Synthetic Construct

<400> 25

Met Asp Tyr Lys Asp Asp Asp Asp Lys Asp Pro Pro Ile Ser Arg Gly
1               5                   10                  15

Ser Glu Phe Leu Asp Asn Lys His Pro Trp Lys Asn Gln Leu Ser Glu
            20                  25                  30

Met Val Gln Pro Ser Gly Gly Pro Ala Glu Asp Gln Asp Met Leu Gly
        35                  40                  45

Glu Glu Ser Ser Leu Gly Lys Pro Ala Met Leu His Leu Pro Ser Glu
    50                  55                  60

Gln Gly Thr Pro Glu Thr Leu Gln Arg Cys Leu Glu Glu Asn Gln Glu
65                  70                  75                  80

Leu Arg Asp Ala Ile Arg Gln Ser Asn Gln Met Leu Arg Glu Arg Cys
                85                  90                  95

Glu Glu Leu Leu His Phe Gln Val Ser Gln Arg Glu Glu Lys Glu Phe
            100                 105                 110

Leu Met Cys Lys Phe Gln Glu Ala Arg Lys Leu Val Glu Arg Leu Ser
        115                 120                 125

Leu Glu Lys Leu Asp Leu Arg Ser Gln Arg Glu Gln Ala Leu Lys Glu
    130                 135                 140

Leu Glu Gln Leu Lys Lys Cys Gln Gln Gln Met Ala Glu Asp Lys Ala
145                 150                 155                 160

Ser Val Lys Val Gln Val Thr Ser Leu Leu Gly Glu Leu Gln Glu Ser
                165                 170                 175

Gln Ser Arg Leu Glu Ala Ala Thr Lys Asp Arg Gln Ala Leu Glu Gly
            180                 185                 190

Arg Ile Arg Ala Val Ser Glu Gln Val Arg Gln Leu Glu Ser Glu Arg
        195                 200                 205

Glu Val Leu Gln Gln Gln His Ser Val Gln Val Asp Gln Leu Arg Met
    210                 215                 220

Gln Asn Gln Ser Val Glu Ala Ala Leu Arg Met Gly Arg Gln Ala Ala
225                 230                 235                 240

Ser Glu Glu Lys Arg Lys Leu Ala Gln Leu Gln Ala Ala Tyr His Gln
                245                 250                 255

Leu Phe Gln Asp Tyr Asp Ser His Ile Lys Ser Asn Lys Gly Met Gln

            260                 265                 270

Leu Glu Asp Leu Arg Gln Gln Leu Gln Gln Ala Glu Glu Ala Leu Val
        275                 280                 285

Ala Lys Gln Glu Leu Ile Asp Lys Leu Lys Glu Glu Ala Glu Gln His
    290                 295                 300

Lys Ile Val Met Glu Thr Val Pro Val Leu Lys Ala Gln Ala Asp Ile
305                 310                 315                 320

Tyr Lys Ala Asp Phe Gln Ala Glu Arg His Ala Arg Glu Lys Leu Val
                325                 330                 335

Glu Lys Lys Glu Tyr Leu Gln Glu Gln Leu Glu Gln Leu Gln Arg Glu
            340                 345                 350

Phe Asn Lys Leu Lys Val Gly Cys His Glu Ser Ala Arg Ile Glu Asp
        355                 360                 365

Met Arg Lys Arg His Val Glu Thr Pro Gln Pro Thr Leu Leu Pro Ala
    370                 375                 380

Pro Ala His His Ser Phe His Leu Ala Leu Ser Asn Gln Arg Arg Ser
385                 390                 395                 400

Pro Pro Glu Glu Pro Pro Asp Phe Cys Cys Pro Lys Cys Gln Tyr Gln
                405                 410                 415

Ala Pro Asp Met Asp Thr Leu Gln Ile His Val Met Glu Cys Ile Glu
            420                 425                 430

<210> 26
<211> 3541
<212> DNA
<213> Artificial

<220>
<223> pcDNA3-GFP-NEMO

<220>
<221> CDS
<222> (1000)..(2964)

<400> 26
gacggatcgg gagatctccc gatcccctat ggtcgactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gcttgcttac 900

atttgcttct gacacaactg tgttcactag caacctcaaa cagacaccat gagatcttat 960

ccatatgatg tgccagatta tgcagccatg gccggatcc atg gtg agc aag ggc 1014
Met Val Ser Lys Gly
1               5

gag gag ctg ttc acc ggg gtg gtg ccc atc ctg gtc gag ctg gac ggc 1062
Glu Glu Leu Phe Thr Gly Val Val Pro Ile Leu Val Glu Leu Asp Gly
                10                  15                  20

gac gta aac ggc cac aag ttc agc gtg tcc ggc gag ggc gag ggc gat 1110
Asp Val Asn Gly His Lys Phe Ser Val Ser Gly Glu Gly Glu Gly Asp
            25                  30                  35

gcc acc tac ggc aag ctg acc ctg aag ttc atc tgc acc acc ggc aag 1158
Ala Thr Tyr Gly Lys Leu Thr Leu Lys Phe Ile Cys Thr Thr Gly Lys
        40                  45                  50

ctg ccc gtg ccc tgg ccc acc ctc gtg acc acc ctg acc tac ggc gtg 1206
Leu Pro Val Pro Trp Pro Thr Leu Val Thr Thr Leu Thr Tyr Gly Val
    55                  60                  65

cag tgc ttc agc cgc tac ccc gac cac atg aag cag cac gac ttc ttc 1254
Gln Cys Phe Ser Arg Tyr Pro Asp His Met Lys Gln His Asp Phe Phe
70                  75                  80                  85

aag tcc gcc atg ccc gaa ggc tac gtc cag gag cgc acc atc ttc ttc 1302
Lys Ser Ala Met Pro Glu Gly Tyr Val Gln Glu Arg Thr Ile Phe Phe
                90                  95                  100

aag gac gac ggc aac tac aag acc cgc gcc gag gtg aag ttc gag ggc 1350
Lys Asp Asp Gly Asn Tyr Lys Thr Arg Ala Glu Val Lys Phe Glu Gly
            105                 110                 115

gac acc ctg gtg aac cgc atc gag ctg aag ggc atc gac ttc aag gag 1398
Asp Thr Leu Val Asn Arg Ile Glu Leu Lys Gly Ile Asp Phe Lys Glu
        120                 125                 130

gac ggc aac atc ctg ggg cac aag ctg gag tac aac tac aac agc cac 1446
Asp Gly Asn Ile Leu Gly His Lys Leu Glu Tyr Asn Tyr Asn Ser His
    135                 140                 145

aac gtc tat atc atg gcc gac aag cag aag aac ggc atc aag gtg aac 1494
Asn Val Tyr Ile Met Ala Asp Lys Gln Lys Asn Gly Ile Lys Val Asn
150                 155                 160                 165

ttc aag atc cgc cac aac atc gag gac ggc agc gtg cag ctc gcc gac 1542
Phe Lys Ile Arg His Asn Ile Glu Asp Gly Ser Val Gln Leu Ala Asp
                170                 175                 180

cac tac cag cag aac acc ccc atc ggc gac ggc ccc gtg ctg ctg ccc 1590
His Tyr Gln Gln Asn Thr Pro Ile Gly Asp Gly Pro Val Leu Leu Pro
            185                 190                 195

gac aac cac tac ctg agc acc cag tcc gcc ctg agc aaa gac ccc aac 1638
Asp Asn His Tyr Leu Ser Thr Gln Ser Ala Leu Ser Lys Asp Pro Asn
        200                 205                 210

gag aag cgc gat cac atg gtc ctg ctg gag ttc gtg acc gcc gcc ggg 1686
Glu Lys Arg Asp His Met Val Leu Leu Glu Phe Val Thr Ala Ala Gly
    215                 220                 225

atc act ctc ggc atg gac gag ctg tac aag gaa ttc ctc gac aac aag 1734
Ile Thr Leu Gly Met Asp Glu Leu Tyr Lys Glu Phe Leu Asp Asn Lys
230                 235                 240                 245

cac ccc tgg aag aac cag ctg agt gag atg gtg cag ccc agt ggt ggc 1782
His Pro Trp Lys Asn Gln Leu Ser Glu Met Val Gln Pro Ser Gly Gly
                250                 255                 260

cca gca gag gac cag gac atg ctg ggt gaa gaa tct tct ctg ggg aag 1830
Pro Ala Glu Asp Gln Asp Met Leu Gly Glu Glu Ser Ser Leu Gly Lys
            265                 270                 275

cct gca atg cta cat ctg cct tca gag cag ggt act cct gag acc ctc 1878
Pro Ala Met Leu His Leu Pro Ser Glu Gln Gly Thr Pro Glu Thr Leu
        280                 285                 290

cag cgc tgc ctg gaa gag aat caa gag ctc cga gac gct atc cgg cag 1926
Gln Arg Cys Leu Glu Glu Asn Gln Glu Leu Arg Asp Ala Ile Arg Gln
    295                 300                 305

agc aat cag atg ctg agg gaa cgc tgt gag gag ctg ctg cat ttc cag 1974
Ser Asn Gln Met Leu Arg Glu Arg Cys Glu Glu Leu Leu His Phe Gln
310                 315                 320                 325

gtc agc cag cgg gag gag aag gag ttc ctt atg tgc aaa ttc cag gaa 2022
Val Ser Gln Arg Glu Glu Lys Glu Phe Leu Met Cys Lys Phe Gln Glu
                330                 335                 340

gcc cgg aag ctg gtg gag aga ctg agc ttg gag aag ctt gat ctt cgg 2070
Ala Arg Lys Leu Val Glu Arg Leu Ser Leu Glu Lys Leu Asp Leu Arg
            345                 350                 355

agt cag agg gaa cag gcc tta aag gag ttg gag caa ctg aag aaa tgc 2118
Ser Gln Arg Glu Gln Ala Leu Lys Glu Leu Glu Gln Leu Lys Lys Cys
        360                 365                 370

caa cag cag atg gct gag gac aag gcc tct gtg aaa gtt cag gtg aca 2166
Gln Gln Gln Met Ala Glu Asp Lys Ala Ser Val Lys Val Gln Val Thr
    375                 380                 385

tca ttg ctc gga gaa ctc cag gag agc cag agc cgt ttg gag gct gcc 2214
Ser Leu Leu Gly Glu Leu Gln Glu Ser Gln Ser Arg Leu Glu Ala Ala
390                 395                 400                 405

acc aag gat cgg caa gct tta gag gga agg att cga gca gtt agt gag 2262
Thr Lys Asp Arg Gln Ala Leu Glu Gly Arg Ile Arg Ala Val Ser Glu
                410                 415                 420

cag gtc aga cag ctg gag agt gag cgg gag gtg cta cag cag cag cac 2310
Gln Val Arg Gln Leu Glu Ser Glu Arg Glu Val Leu Gln Gln Gln His
            425                 430                 435

agc gtc cag gtg gac cag ctg cgt atg cag aac cag agc gtg gag gct 2358
Ser Val Gln Val Asp Gln Leu Arg Met Gln Asn Gln Ser Val Glu Ala
        440                 445                 450

gcc ttg cga atg ggg cgg cag gct gct tca gag gag aag cgg aag ctg 2406
Ala Leu Arg Met Gly Arg Gln Ala Ala Ser Glu Glu Lys Arg Lys Leu
    455                 460                 465

gct cag ttg cag gca gcc tat cac cag ctc ttc caa gac tac gac agc 2454
Ala Gln Leu Gln Ala Ala Tyr His Gln Leu Phe Gln Asp Tyr Asp Ser
470                 475                 480                 485

cac att aag agc aac aag ggc atg cag ctg gaa gat ctg agg caa cag 2502
His Ile Lys Ser Asn Lys Gly Met Gln Leu Glu Asp Leu Arg Gln Gln
                490                 495                 500

ctc cag caa gct gag gag gcc ctg gta gcc aaa cag gaa ttg att gat 2550
Leu Gln Gln Ala Glu Glu Ala Leu Val Ala Lys Gln Glu Leu Ile Asp
            505                 510                 515

aag ctg aaa gag gag gct gag cag cac aag att gtg atg gag act gtg 2598
Lys Leu Lys Glu Glu Ala Glu Gln His Lys Ile Val Met Glu Thr Val
        520                 525                 530

cca gtc ttg aag gcc cag gcg gat atc tac aag gct gac ttc caa gct 2646
Pro Val Leu Lys Ala Gln Ala Asp Ile Tyr Lys Ala Asp Phe Gln Ala
    535                 540                 545

gag agg cat gcc cgg gag aag ctg gtg gag aag aag gag tat ttg cag 2694
Glu Arg His Ala Arg Glu Lys Leu Val Glu Lys Lys Glu Tyr Leu Gln
550                 555                 560                 565

gag cag ctg gag cag ctg cag cgc gag ttc aac aag ctg aaa gtt ggc 2742
Glu Gln Leu Glu Gln Leu Gln Arg Glu Phe Asn Lys Leu Lys Val Gly
                570                 575                 580

tgc cat gag tca gcc agg att gag gat atg agg aag cgg cat gta gag 2790
Cys His Glu Ser Ala Arg Ile Glu Asp Met Arg Lys Arg His Val Glu
            585                 590                 595

act ccc cag cct act tta ctc cct gct cca gct cac cac tcc ttt cat 2838
Thr Pro Gln Pro Thr Leu Leu Pro Ala Pro Ala His His Ser Phe His
        600                 605                 610

ttg gcc ttg tcc aac cag cgg agg agc cct cct gaa gaa cct cct gac 2886
Leu Ala Leu Ser Asn Gln Arg Arg Ser Pro Pro Glu Glu Pro Pro Asp
    615                 620                 625

ttc tgt tgt ccg aag tgc cag tat cag gct cct gat atg gac act cta 2934
Phe Cys Cys Pro Lys Cys Gln Tyr Gln Ala Pro Asp Met Asp Thr Leu
630                 635                 640                 645

cag ata cat gtc atg gag tgc ata gag tag gggcagcaga tgcaaggcca 2984
Gln Ile His Val Met Glu Cys Ile Glu
                650

cttgcagtac tatgtcctga tctgtgtgac ttgtgctttc ctgttttacc tgcatagtcc 3044

acacttaagg gcttgcttta gccctttggt cccccattta gggtagacag ccccattcag 3104

ggcttttttt tttttctgtg tgcctgatcc agtttgcctc tggtggcttc ttccctctct 3164

cccatagtcc tagggagtct agagggccct attctatagt gtcacctaaa tgctagagct 3224

cgctgatcag cctcgactgt gccttctagt tgccagccat ctgttgtttg cccctccccc 3284

gtgccttcct tgaccctgga aggtgccact cccactgtcc tttcctaata aaatgaggaa 3344

attgcatcgc attgtctgag taggtgtcat tctattctgg ggggtggggt ggggcaggac 3404

agcaaggggg aggattggga agacaatagc aggcatgctg gggatgcggt gggctctatg 3464

gcttctgagg cggaaagaac cagctggggc tctagggggt atccccacgc gccctgtagc    3524
ggcgcattaa gcgcggc                                                  3541

<210>  27
<211>  654
<212>  PRT
<213>  Artificial

<220>
<223>  Synthetic Construct

<400>  27

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Glu
225                 230                 235                 240

Phe Leu Asp Asn Lys His Pro Trp Lys Asn Gln Leu Ser Glu Met Val
                245                 250                 255

Gln Pro Ser Gly Gly Pro Ala Glu Asp Gln Asp Met Leu Gly Glu Glu
            260                 265                 270

Ser Ser Leu Gly Lys Pro Ala Met Leu His Leu Pro Ser Glu Gln Gly
        275                 280                 285

Thr Pro Glu Thr Leu Gln Arg Cys Leu Glu Glu Asn Gln Glu Leu Arg
    290                 295                 300

Asp Ala Ile Arg Gln Ser Asn Gln Met Leu Arg Glu Arg Cys Glu Glu
305                 310                 315                 320

Leu Leu His Phe Gln Val Ser Gln Arg Glu Glu Lys Glu Phe Leu Met
                325                 330                 335

Cys Lys Phe Gln Glu Ala Arg Lys Leu Val Glu Arg Leu Ser Leu Glu
            340                 345                 350

Lys Leu Asp Leu Arg Ser Gln Arg Glu Gln Ala Leu Lys Glu Leu Glu
        355                 360                 365

Gln Leu Lys Lys Cys Gln Gln Gln Met Ala Glu Asp Lys Ala Ser Val
    370                 375                 380

Lys Val Gln Val Thr Ser Leu Leu Gly Glu Leu Gln Glu Ser Gln Ser
385                 390                 395                 400

Arg Leu Glu Ala Ala Thr Lys Asp Arg Gln Ala Leu Glu Gly Arg Ile
                405                 410                 415

Arg Ala Val Ser Glu Gln Val Arg Gln Leu Glu Ser Glu Arg Glu Val
            420                 425                 430

Leu Gln Gln Gln His Ser Val Gln Val Asp Gln Leu Arg Met Gln Asn
        435                 440                 445

Gln Ser Val Glu Ala Ala Leu Arg Met Gly Arg Gln Ala Ala Ser Glu
    450                 455                 460

Glu Lys Arg Lys Leu Ala Gln Leu Gln Ala Ala Tyr His Gln Leu Phe
465                 470                 475                 480

Gln Asp Tyr Asp Ser His Ile Lys Ser Asn Lys Gly Met Gln Leu Glu
                485                 490                 495

Asp Leu Arg Gln Gln Leu Gln Gln Ala Glu Glu Ala Leu Val Ala Lys
            500                 505                 510

Gln Glu Leu Ile Asp Lys Leu Lys Glu Glu Ala Glu Gln His Lys Ile
        515                 520                 525

Val Met Glu Thr Val Pro Val Leu Lys Ala Gln Ala Asp Ile Tyr Lys
    530                 535                 540

Ala Asp Phe Gln Ala Glu Arg His Ala Arg Glu Lys Leu Val Glu Lys
545                 550                 555                 560

Lys Glu Tyr Leu Gln Glu Gln Leu Glu Gln Leu Gln Arg Glu Phe Asn
                565                 570                 575

Lys Leu Lys Val Gly Cys His Glu Ser Ala Arg Ile Glu Asp Met Arg
            580                 585                 590

Lys Arg His Val Glu Thr Pro Gln Pro Thr Leu Leu Pro Ala Pro Ala
        595                 600                 605

His His Ser Phe His Leu Ala Leu Ser Asn Gln Arg Arg Ser Pro Pro
    610                 615                 620

Glu Glu Pro Pro Asp Phe Cys Cys Pro Lys Cys Gln Tyr Gln Ala Pro
625                 630                 635                 640

Asp Met Asp Thr Leu Gln Ile His Val Met Glu Cys Ile Glu
                645                 650

## Description

The present invention relates to a method for identifying and selecting molecules that modulate (activate or inhibit) the NF-κB pathway activation by modulating the interaction of NEMO with other proteins.

Nuclear factor-KB (NF-κB) signaling is a signal transduction pathway involved in a variety of essential cellular processes including inflammatory responses, oncogenesis, viral infection, regulation of cell proliferation, apoptosis and antigenic stimulation of B and T lymphocytes (Ghosh, 1998, Annu. Rev. Immunol.; Karin, 1999, J. Biol. Chem.; Israel, 2000, Trends Cell Biol.; Santoro, 2003, EMBO J.). In mammalian cells, there are five NF-κB family members that dimerize: RelA, RelB, c-Rel, NF-κB2/p100/p52 and NF-κB1/p105/p50. NF-κB whose predominant form is a heterodimeric transcription factor composed of p50 and RelA subunits, remains, in resting cells, sequestered in the cytoplasm through their association with members of an inhibitory family of proteins known as IκB. Upon stimulation by several factors, including the cytokines TNF-α and the interleukin-1, endotoxin (LPS), microbial and viral infections, pro-inflammatory signals converge on the canonical IκB kinase complex (IKK), a protein complex that is composed of two kinases subunits, IKKα/IKK-1 and IKKβ/IKK-2 and a structural/regulatory subunit NEMO/IKK-γ. Once activated IKK complex phosphorylates IκB proteins, triggering their ubiquitination and subsequent degradation by proteasome. The released NF-κB transcription factors are then translocated into the nucleus to initiate or up-regulate gene expression. Although IKKα and IKKβ exhibit striking structural similarity (52%), exquisite genetic studies have shown that they are involved in two pathways for the activation of NF-κB (Pomerantz, 2002, Mol Cell). IKKβ is the pro-inflammatory kinase that is responsible of activation of classical NF-κB complexes whereas IKKα in association with NF-κB inducing kinase (NIK) plays essential roles in the non-canonical NF-κB signaling pathway (Senftleben, 2001, Science). IKKα plays also a role in keratinocyte differentiation but this process is independent of its kinase activity (Hu, 2001, Nature).

The NEMO protein (NF-κB essential modulator) plays a key role in the NF-κB pathway activation. The NEMO protein is associated with IKKα and IKKβ protein kinases in the IKK complex. The IKK kinases are activated by phosphorylation upon an unknown mechanism, which is believed to be a result of NEMO oligomerization (Agou et al., 2004, J. Biol. Chem.). The presence of the NEMO protein underlies IKK activation since NEMO-deficient cells are unable to activate NF-κB in response to many stimuli.

The biochemical mechanisms triggering the activation of IKK in response to pro-inflammatory stimuli remain unclear. It has been demonstrated that phosphorylation on two serine residues in the activation T-loop induces activation of the IKKβ. However, the mechanism that leads to this phosphorylation event is still unknown. One possible mechanism consists of the conformation change of the kinase induced by NEMO oligomerization (Agou et al., 2004, J. Biol. Chem.). This change of the oligomeric state may induce the T-loop activation by a mechanism of transautophosphorylation (Zandi, 1997, Cell; Tang, 2003, J. Biol. Chem.). Consistent with the role of NEMO oligomerization in IKK activation, mutations in the minimal oligomerization domain failed to rescue NF-κB by genetic complementation in NEMO-deficient cells activation in responses to many stimuli. Moreover, enforced oligomerization of NEMO lead to full activation of IKK complex. (Inohara, 2000, J. Biol. Chem.; Poyet, 2000, J. Biol. Chem.; Poyet, 2001, J. Biol. Chem.). Recently, the phosphorylation and the ubiquitination of NEMO in response to TNF-α have been reported, (Carter, 2001, J. Biol. Chem.; Trompouky, 2003, Nature; Kovalenko, 2003, Nature). However, these NEMO modifications have not been demonstrated yet as a crucial step to activate IKK complex in response to several pro-inflammatory stimuli.

Inhibition of NF-κB activation constitutes a privileged target for development of new anti-inflammatory and anti-cancer drugs (May, 2000, Science; Poulaki, 2002, Am J Pathol..). Among many protein actors in NF-κB signaling pathway, IKK complex represents one of the most promising molecular targets for discoveries of new specific NF-κB inhibitors. To minimize the potential toxicity effects *in vivo,* therapeutical success will greatly depend on the abilities of the NF-κB inhibitors to block activating signals without modifying the basal level of NF-κB activity. May *et al.* described a cell-permeable peptidic inhibitor that block specifically the pro-inflammatory NF-κB activation by disrupting the constitutive NEMO interaction with IKK kinases (May, 2000, Science; May, 2002, J. Biol. Chem.). Modulating protein-protein interactions by the rational design of peptide that alter protein's function provides an important tool for both basic research and development of new classes of therapeutic drugs (Souroujon, 1998, Nat Biotechnol.), especially with signaling proteins that exhibit flexible and dynamic binding properties (Pawson, 2003, Science). Numerous studies of peptide modulators have been described in the literature where peptides mediate protein's function by interfering with localization (translocation) (Lin, 1995, J. Biol. Chem.), recruitment to receptor (Chang, 2000, J. Biol. Chem.), intramolecular interactions (Souroujon, 1998, Nat Biotechnol.) and oligomerization (Judice, 1997, P.N.A.S.). In the latter, inhibition of HIV-1 gp41 fusion protein with various peptides provides a clear proof-of concept (for a review see Chan, 1998, Cell and Eckert, 2001, Ann. Rev. Biochem.).

Therefore, the NEMO protein is a promising target for the development of new drugs inhibiting the NF-κB pathway, since it integrates and coordinates most of NF-κB stimuli and it is a non redundant component of the IκB Kinase complex (IKK).

The amino acid sequence of NEMO protein suggests that said protein consists of several domains (Agou et al., J. Biol. Chem., 2004b). Briefly, the N-terminal part of the polypeptide contains a large coiled-coil motif (CC1) and all the residues involved in the interaction of the protein with the IKK kinases (IKK-binding domain). The C-terminal half (residues 250-412) is composed of two successive coiled-coil motifs, CC2 (residues 253-285) and LZ (residues 301-337), and a zinc finger motif (ZF) at the extreme C-terminus of the protein and functions as the regulatory part of the protein, which has often been reported as a binding template to link many upstream signaling molecules or viral proteins (Ghosh, 1998, Annu. Rev. Immunol.; Santoro, 2003, EMBO J.). Interestingly, mutations responsible for incontinentia pigmenti (IP) and ectodermal dysplasia with immunodeficiency (EDA-ID) were mainly found in this part of the molecule (Döffinger, 2001, Nature Gen.; Zonana, 2000, Am. J. Hum. Genet). The minimal oligomerization domain (MOD) of NEMO consists of the CC2 and LZ coiled-coil motifs (Agou et al., J. Biol. Chem., 2004a). The MOD domain also includes a NLM motif (Nemo Like Motif) (293-322) (Agou et al., 2004b). The residues numeration indicated above is relative to the murine NEMO protein of sequence SEQ ID NO: 1. With respect to the human NEMO protein, CC2, LZ and NLM domains respectively correspond to residues 260-292, residues 301-344 and residues 300-329 of the sequence SEQ ID NO: 2.

The Inventors have previously synthesized peptides, derived from the MOD domain of NEMO, which are able to inhibit the NF-κB pathway by inhibiting the NEMO oligomerization (Patent Application WO 2005/027959). Indeed, spLZ peptides of sequences SEQ ID NO: 6 and SEQ ID NO: 7, which respectively correspond to the LZ domain of the murine and the human NEMO proteins, and spCC2 peptides of sequences SEQ ID NO: 8 and SEQ ID NO: 9, which respectively correspond to the CC2 domain of the murine and the human NEMO proteins, inhibit NF-κB activation with an IC₅₀ in the µM range (WO2005/027959, Agou *et al.,* 2004b).

Other peptides, NLM and DR-NLM, deriving from the N-terminal sequence of the LZ motif of NEMO also inhibit NF-κB activation. Their IC₅₀ values were reported in the International PCT Application W0 2005/027959. The DR-NLM peptide is half the length of the LZ peptide (International PCT Application WO 2005/027959). Peptides NLM (SEQ ID NO: 10) and DR NLM (SEQ ID NO: 11) correspond to residues 294-314 of SEQ ID NO: 1 and differ in that in DR NLM the Asp residue in position 304 of SEQ ID NO: 1 is substituted with a Arg residue. NLM and DR NLM derived from human NEMO protein are respectively defined by SEQ ID NO: 18 and SEQ ID NO: 12.

Unexpectedly, the Inventors have now found that the MOD domain acts as oligomerization domain as well as polyubiquitin chain binding domain. These dual properties of said NEMO domains allow to search for new compounds modulating specifically the NF-κB signaling pathway by modulating (enhancing or disrupting) NEMO oligomerization or by modulating (enhancing or disrupting) the interaction between NEMO and the polyubiquitin chain. Indeed, whilst the LZ specifically inhibits the NF-κB activation by disrupting NEMO oligomerization, the DR NLM acts as NF-κB inhibitor by blocking the interaction between the polyubiquitin chains and the NEMO protein. These results also provide new insights into the NLM domain of NEMO which corresponds to a new and crucial ubiquitin binding domain required for the NEMO function. More importantly, these results allow to define the "hot spot" region of NEMO which could be used as a target for virtual screening.

Indeed, considering the role of NEMO in inflammatory response, oncogenesis and viral infection and the complexity of the NF-κB signaling pathway, there is a need of primary screening of substances able to act by disrupting any NEMO interaction with other proteins (*i.e.* oligomerization or interaction between NEMO and the polyubiquitin chain).

Therefore, the purpose of the present invention is to provide a screening assay for selecting molecules that specifically modulate (activate or inhibit) the interaction of NEMO with other proteins. More specifically, said screening assay uses the MOD domain of the NEMO, for detecting new efficient and specific modulators of the NF-κB pathway activation.

Accordingly, a first object of the present invention is a method for primary screening for a potential modulator of the NF-κB pathway activation, characterized in that it comprises the following steps:
(a) bringing into contact (i) a peptide P1 comprising the CC2 and LZ regions of NEMO, said peptide P1 being labelled or not, and (ii) a peptide P2, labelled or not, selected from the group consisting of a peptide comprising at least 10 amino acid residues of the LZ region of NEMO, preferably at least 36 amino acid residues of the LZ region of NEMO, a peptide comprising at least 10 amino acid residues of the NLM region of NEMO, preferably at least 21 amino acid residues of the NLM region of NEMO, a peptide comprising DR-NLM, a peptide comprising the regions CC2 and LZ of NEMO, a peptide comprising at least 10 amino acids residues of the CC2 region of NEMO, and a peptide comprising a K63-linked polyubiquitinylated chain; in the absence of the substance S to be tested;
(b) bringing into contact said peptide P1, labelled or not, and said peptide P2, labelled or not, in the presence of the substance S to be tested;
(c) detecting the complexes between P1 and P2 obtained in step (a) by measuring an appropriate signal;
(d) detecting the complexes between P1 and P2 obtained in step (b) by measuring an appropriate signal;
(e) comparing the signal measured in (c) and the signal measured in (d) and selecting the substance S for which the ratio of the signal measured in (c)/signal measured in (d) is different from 1.

According to the present invention, the terms region(s) and domain(s) are interchangeable. The CC2 and LZ regions (or domains) of NEMO are as described above.

Preferably, P1 is selected from the group consisting of the peptide sp CC2-LZ of SEQ ID NO: 17, the peptide spCC2-LZ of SEQ ID NO: 19, the murine NEMO protein of SEQ ID NO: 1, and the human NEMO protein of SEQ ID NO: 2.

Preferably, P2 is selected from the group consisting of the peptides of sequences SEQ ID NO: 1, SEQ ID NO: 2, SEQ ID NO: 6 to 12, and SEQ ID NO: 17 to 19.

The peptides used in the method according to the invention can be synthesized as described in the Patent Application WO 2005/027959 and in Agou *et al.,* 2004b.

The prefix "sp" (synthetic polypeptide) means that said peptide is synthetic and may be used for instance to distinguish a peptide (*e.g.* spCC2) from the corresponding region of the NEMO protein (*e.g.* CC2 region). However, peptide and sp peptide refer to the same product.

Furthermore, without precision, the different peptides relate indifferently to the murine NEMO protein and to the human NEMO protein.

Preferably, said K63-linked polyubiquitinylated molecule is selected from the group consisting of a K63-linked polyubiquitine chain, for example K63Ub₂₋₇ (Boston Biochem, Inc), a protein coupled to a K63-linked polyubiquitine chain, such as the polyubiquitinylated protein RIP1 (Receptor Interacting Protein kinase 1) (Ea *et al.,* 2006), and a cell extract comprising K63-linked polyubiquitinylated proteins, such as a cell extract from JM4.5.2 cells which have been previously stimulated with TNF-α.

In a preferred embodiment of said method, said peptide P1 is immobilized on a solid support. Preferably, said solid support consists of magnetic beads. Advantageously, the peptide P1 is coupled to a biotinyl group and is immobilized on streptavidin magnetic beads.

Preferably, the signal in steps (c) and (d) is measured by Western blot, by fluorescent anisotropy, by Fluorescent Resonance Energy Transfer (FRET) or by Homogeneous Time Resolved Fluorescence (HTRF).

In a preferred embodiment of said method, said peptide P1 is unlabelled, and said peptide P2 is labelled with a fluorescent marker M1.

Preferably, said marker M1 is selected from the group consisting of: green fluorescent protein (GFP), cyan fluorescent protein, yellow fluorescent protein and a fluorophore comprising a maleimide group. Other examples of fluorescent markers are described in Shaner et al. (Nat. Biotech., 2004, 22, 1567). Other fluorophores that can be used include CFP/ds Red and/or GFP/ds Red (Erickson et al., Biophys J., 2003, 85, 599-611) and Cyan variants of the orange type (Karawawa et al., Biochem J., 2004, 381(Pt1), 307-12). Example of a fluorescent marker comprising a maleimide group is the Bodipy^{®}FL N-(2-aminoethyl) maleimide fluorophore (Molecular Probe).

Preferably, said labelled peptide P2 is selected from the group consisting of the peptides of sequences SEQ ID NO: 4, 5 and 13 to 16.

In further another preferred embodiment of said method, said peptide P1 is labelled with a marker M2 and said peptide P2 is labelled with a marker M3, M2 and M3 being a couple of a fluorophore donor and a fluorophore acceptor.

Examples of couples of fluorophore donor-fluorophore acceptor are the couple Europium cryptate-XL665 (Cis-Bio) and the couple Cy5/5 (GE Healthcare)-BHQ3 (Biosearch Technologies).

Preferably, a hybrid construct is used such that the peptide P1 and/or P2 is linked, either directly or through a linker molecule (*e.g.*, a short peptide), to the fluorescent protein. The linkage can occur at the N-terminus or C-terminus of the peptide, preferably provided that the added linkage does not interfere with the binding between P1 and P2.

The interaction between P1 and substances that modulate (activate or inhibit) its binding to P2 can be detected for instance using the FRET method, by direct measurement of the fluorescence emission of the fluorophore acceptor resulting from the proximity of the two different markers (fluorophore donor and fluorophore acceptor). A decrease in the fluorescence emission of the fluorophore acceptor in the presence of the substance S, compared to the fluorescence emission of said fluorophore acceptor in the absence of said substance S, indicates that said substance S interferes with the binding of P1 to P2. Hence, a decrease in said fluorescent emission indicates that said substance S interferes with the binding of P1 to P2, while an increase in said fluorescent emission indicates that said substance S enhances said binding of P1 to P2.

Advantageously, M2 consists of a first tag and an antibody directed against said first tag and labelled with a fluorophore donor, and M3 consists of a second tag and an antibody directed against said second tag and labelled with a fluorophore acceptor, said two tags being different.

Preferably, said tag of the marker M2 is a poly His sequence, and said tag of the marker M3 is biotine.

In a further preferred embodiment of said method, said potential modulator is a potential activator and step (e) comprises comparing the signal measured in (c) and the signal measured in (d) and selecting the substance S for which the ratio of the signal measured in (c)/signal measured in (d) is inferior to 1.

In another preferred embodiment of said method, said potential modulator is a potential inhibitor and step (e) comprises comparing the signal measured in (c) and the signal measured in (d) and selecting the substance S for which the ratio of the signal measured in (c)/signal measured in (d) is superior to 1.

In the case where the peptide P2 represents a peptide comprising a K63-linked polyubiquitine chain, the peptide P1 is advantageously selected from the group consisting of a peptide comprising at least 10 amino acid residues of the NLM region of NEMO, preferably at least 21 amino acid residues, and a peptide comprising DR NLM. Preferably, said peptide P1 is selected from the group consisting of the peptides of sequences SEQ ID NO: 1, 2, 10 and 17 to 19.

Thus, in such a case, the method for primary screening for a potential modulator of the NF-κB pathway activation, is characterized in that it comprises the following steps:
(a) bringing into contact (i) a peptide P1 selected from the group consisting of a peptide comprising at least 10 amino acid residues of the NLM region of NEMO, preferably at least 21 amino acid residues, and a peptide comprising DR NLM, said peptide P1 being labelled or not, and (ii) a peptide P2 comprising a K63-linked polyubiquitine chain, said peptide P2 being labelled or not, in the absence of the substance S to be tested;
(b) bringing into contact said peptide P1, labelled or not, and said peptide P2, labelled or not, in the presence of the substance S to be tested;
(c) detecting the complexes between P1 and P2 obtained in step (a) by measuring an appropriate signal;
(d) detecting the complexes between P1 and P2 obtained in step (b) by measuring an appropriate signal;
(e) comparing the signal measured in (c) and the signal measured in (d) and selecting the substance S for which the ratio of the signal measured in (c)/signal measured in (d) is different from 1.

Preferably, said peptide P1 is selected from the group consisting of the peptides of sequences SEQ ID NO: 1, 2, 10 and 17 to 19.

Preferably, said peptide P2, comprising a K63-linked polyubiquitinylated chain, is immobilised on a solid support, as described above.

Preferably, said peptide P2 is labelled with a fluorescent marker M1 as described here above and the signal in steps (c) and (d) is measured as described above.

In a preferred embodiment of said method, said potential modulator is a potential activator and step (e) comprises comparing the signal measured in (c) and the signal measured in (d) and selecting the substance S for which the ratio of the signal measured in (c)/signal measured in (d) is inferior to 1.

In another preferred embodiment of said method, said potential modulator is a potential inhibitor and step (e) comprises comparing the signal measured in (c) and the signal measured in (d) and selecting the substance S for which the ratio of the signal measured in (c)/signal measured in (d) is superior to 1.

Alternatively, the method for primary screening for a potential modulator of the NF-κB pathway activation, comprises the following steps:
(a) bringing into contact a peptide P1 comprising the CC2 and LZ regions of NEMO with the substance S to be tested, said peptide P1 being labelled with the markers M2 and M3, i.e, being coupled to both a fluorophore donor and to a fluoropohore acceptor;
(b) measuring the fluorescence emission in the absence of the substance S to be tested;
(c) measuring the fluorescence emission in the presence of the substance S to be tested;
(d) comparing the signal measured in (b) and the signal measured in (c) and selecting the substance S for which the ratio signal measured in (b)/signal measured in (c) is different from 1.

Preferably, said signal in steps (c) and (d) is measured by FRET or by HTRF as described above.

Advantageously, said fluorophore donor is coupled to the N-terminus of the CC2 region, and said fluorophore acceptor is coupled to the C-terminus of the LZ region.

In a preferred embodiment of said alternative method, said potential modulator is a potential activator and step (d) comprises comparing the signal measured in (b) and the signal measured in (c) and selecting the substance S for which the ratio of the signal measured in (b)/signal measured in (c) is inferior to 1.

In another preferred embodiment of said alternative method, said potential modulator is a potential inhibitor and step (d) comprises comparing the signal measured in (b) and the signal measured in (c) and selecting the substance S for which the ratio of the signal measured in (b)/signal measured in (c) is superior to 1.

Alternatively, the method for primary screening for a potential modulator of NF-κB pathway activation is characterized in that it comprises the following steps:
a) providing a cell deficient in endogenous NEMO, which is transformed with one or more polynucleotides that express at least a peptide P1 and a peptide P2 as defined in the first object of the present invention;
b) applying the substance S to the cell;
c) detecting the formation of complexes between P1 and P2 in the absence of the substance S by measuring an appropriate signal;
d) detecting the formation of complexes between P1 and P2 in the presence of the substance S by measuring an appropriate signal;
e) comparing the signal measured in (c) and the signal measured in (d) and selecting the substance S for which the ratio of the signal measured in (c)/signal measured in (d) is different from 1.

In a preferred embodiment of this alternative method, P1 is a fusion protein GFP-NEMO and P2 is a fusion protein Flag-NEMO, Flag being a hydrophilic 8 amino acid residues peptide (SEQ ID NO: 23). Preferably, P1 is defined by the sequence SEQ ID NO: 27 and P2 is defined by the sequence SEQ ID NO: 25.

In another preferred embodiment of this alternative method, the steps (c) and d) comprise:
c1) (or d1)) the cell lysis,
c2) (or d2)) the immunoprecipitation of Flag-NEMO, bound or not to GFP-NEMO, using antibody directed against the Flag moiety of Flag-NEMO, and
c3) (or d3)) the detection of the fluorescence emission of GFP moiety of GFP-NEMO bound to the immunoprecipitated Flag-NEMO.

In a preferred embodiment of said alternative method, said potential modulator is a potential activator and step (e) comprises comparing the signal measured in (c) and the signal measured in (d) and selecting the substance S for which the ratio of the signal measured in (c)/signal measured in (d) is inferior to 1.

In another preferred embodiment of said alternative method, said potential modulator is a potential inhibitor and in that step (e) comprises comparing the signal measured in (c) and the signal measured in (d) and selecting the substance S for which the ratio of the signal measured in (c)/signal measured in (d) is superior to 1.

Another object of the present invention is a method for secondary screening for an inhibitor of NF-κB pathway activation, comprising:
a) selecting a substance S potentially able to inhibit NF-κB pathway activation by any method for primary screening for a potential inhibitor according to the invention as described above,
b) applying an activator of NF-κB pathway in the absence or in the presence of the substance S selected in a), to a cell comprising a reporter gene under the control of a promoter inducible by NF-κB;
c) detecting the expression of the reporter gene by measuring an appropriate signal in the absence of the substance S;
d) detecting the expression of the reporter gene by measuring an appropriate signal in the presence of the substance S; and
e) comparing the signal measured in (c) and the signal measured in (d) and selecting the substance for which the ratio of the signal measured in (c)/signal measured in (d) is superior to 1.

Preferably, said activator of NF-κB pathway used in step c) is selected from the group consisting of: TNF-α, IL-1β, PMA, ionomycin, and LPS from *Salmonella abortus.*

The reporter gene is preferably selected from the group consisting of the *lacZ* gene, encoding the β-galactosidase enzyme, and the *luc* gene, encoding the luciferase. The reporter gene is preferably under the control of a promoter comprising the SRE sequence (Serum Response Element). Such constructs are described in the Patent Application WO2005/027959 and in Agou *et al.,* 2004b.

In a preferred embodiment of this method for secondary screening, said cell is a 70Z/3-C3 cell, deposited in the Collection Nationale de Cultures de Microorganismes (C.N.C.M.) on April 1, 2003, under the number 1-3004.

In another preferred embodiment, said cell is a T lymphocyte or a MEF cell (Murine embryonic Fibroblast) comprising a reporter system for the NF-κB pathway.

It is preferred that said inhibitor of the NF-κB pathway activation, selected by the methods according to the invention, is a specific inhibitor of the NF-κB pathway which does not inhibit the ERK and p38 pathway activation, the NF-AT pathway activation, and the AP1 pathway activation.

Therefore the secondary method for screening advantageously comprises the additional steps of determining whether the selected inhibitor acts on the ERK and p38 pathway, on the NF-AT pathway and on the AP1 pathway. The inhibitors which are selected by said method are those which do not inhibit ERK, p38, NF-AT and AP1 pathways activation.

Assaying the inhibition of the ERK and p38 pathway, the NF-AT pathway and the AP1 pathway may be carried out for instance using a reporter gene placed under the control of a promoter inducible by the corresponding pathway. Examples of reporter genes are described above. Examples of constructs inducible by the ERK and p38 pathway, the NFAT pathway, and the AP1 pathway are respectively SRE-luc (Courtois et al., 1997, Mol. Cell. Biol, 17:1441-1449), NF-AT-luc and AP1-luc (Northrop et al., 1993, J.Biol.Cell., 268:2917-2923). Expression of the reporter gene is assayed for example by using transformed cells, such as comprising the corresponding construct, stimulated by PMA and ionomycin.

Another object of the present invention is a method for secondary screening for an activator of NF-κB pathway activation, comprising:
a) selecting a substance S potentially able to activate NF-κB pathway activation by a method for primary screening for a potential activator according to the invention as described above,
b) providing a cell comprising a reporter gene under the control of a promoter inducible by NF-κB;
c) detecting the expression of the reporter gene by measuring an appropriate signal in the absence of the substance S;
d) detecting the expression of the reporter gene by measuring an appropriate signal in the presence of the substance S; and
e) comparing the signal measured in (c) and the signal measured in (d) and selecting the substance for which the ratio of the signal measured in (c)/signal measured in (d) is inferior to 1.

The reporter gene is preferably selected from the group consisting of the *lacZ* gene, encoding the β-galactosidase enzyme, and the *luc* gene, encoding the luciferase. The reporter gene is preferably under the control of a promoter comprising the SRE sequence (Serum Response Element) as described above.

In a preferred embodiment of this method for secondary screening, said cell is a 70Z/3-C3 cell, deposited in the Collection Nationale de Cultures de Microorganismes (C.N.C.M.) on April 1, 2003, under the number I-3004.

A more complete appreciation of the invention and many of the attendant advantages thereof will be readily obtained as the same becomes better understood by reference to the following Figures in conjunction with the detailed description below.

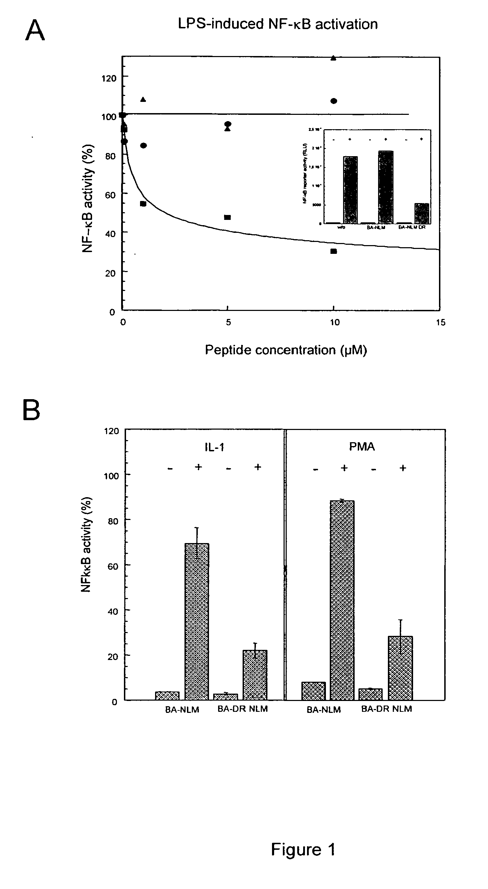

**Figure 1** **: Inhibition of NF-κB activation by the peptide DR NLM. A,** Stably transfected 70Z/3-C3 cells were incubated with bodipy tagged Antennapedia (BA) (black triangles), BA-NLM (filled circles) and BA-DR NLM (filled squares) (0-10 µM) for 2 h before activation with 15 µg/ml LPS for 4 h. NF-κB activity was measured using the β-galactosidase (β-Gal) assay. Inset, NF-κB activity after incubation with or without (w/o) 10 µM of BA-NLM or BA-DR NLM peptides with (+) or without (-) LPS activation. **B,** 70Z/C3 cells were incubated for 2 h with 10 µM of BA-NLM or BA-DR NLM peptides. Cells were treated for 5 h with (+) or without (-) PMA or IL-1. NF-κB activity was measured using the β-galactosidase assay.

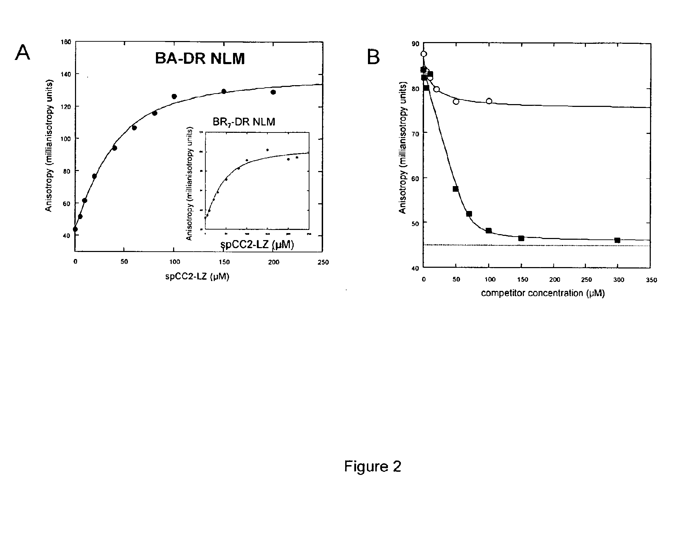

**Figure 2****: *In vitro* binding of the peptide BA-DR NLM to the peptide spCC2-LZ. A**, Analysis of the formation of BA-DR NLM:spCC2-LZ complexes using fluorescent anisotropy. Inset : analysis of the formation of complexes between BR₇-DR NLM and spCC2-LZ using fluorescent anisotropy. **B**, Determination of the binding site of BA-DR NLM to spCC2-LZ by competition assay using fluorescence anisotropy. Fluorescent BA-DR NLM (10 µM) was incubated with spCC2-LZ (30 µM) to form the BA-DR NLM:spCC2-LZ complex. Increasing concentrations of A-NLM (*open circles*) and of the C-terminal part of LZ fused to the antennapedia sequence (A-Cterm-LZ) (*filled squares*) were then added to displace the BA-DR NLM bound to spCC2-LZ. The dashed line represents the anisotropy level of free BA-DR NLM.

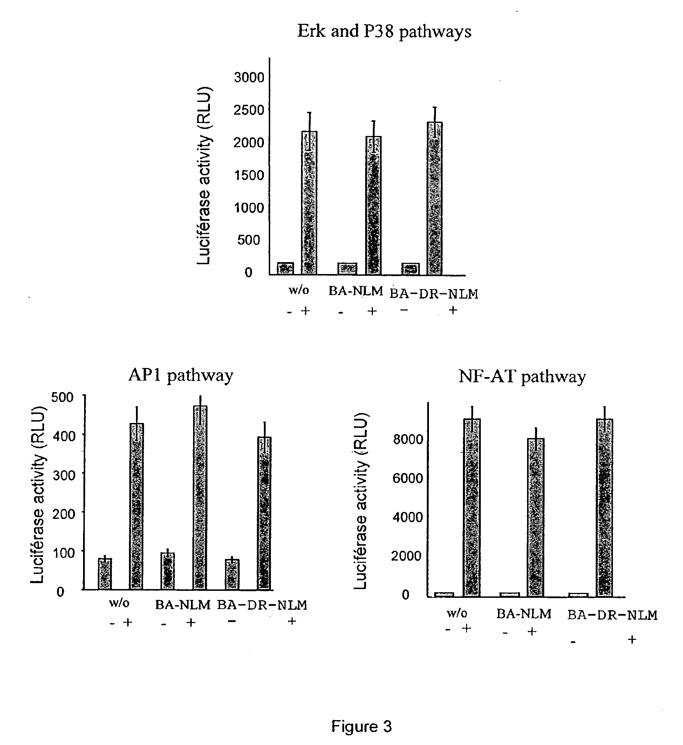

**Figure 3****: the peptide DR NLM does not affect other signalling pathways** Inhibition effect of BA-NLM and BA-DR NLM on the AP1, Erk and P38 MAPK pathways and the NFAT pathway. Jurkat cells transiently transfected with different reporter plasmids (SRE-luc, AP1-luc, NFAT-luc) were incubated with or without (w/o) 5 µM of BA-NLM or BA-DR NLM for 2 h and then mock-stimulated (-) or stimulated (+) with PMA (100 ng/ml) and ionomycin (1 µg/ml) for 5 h.

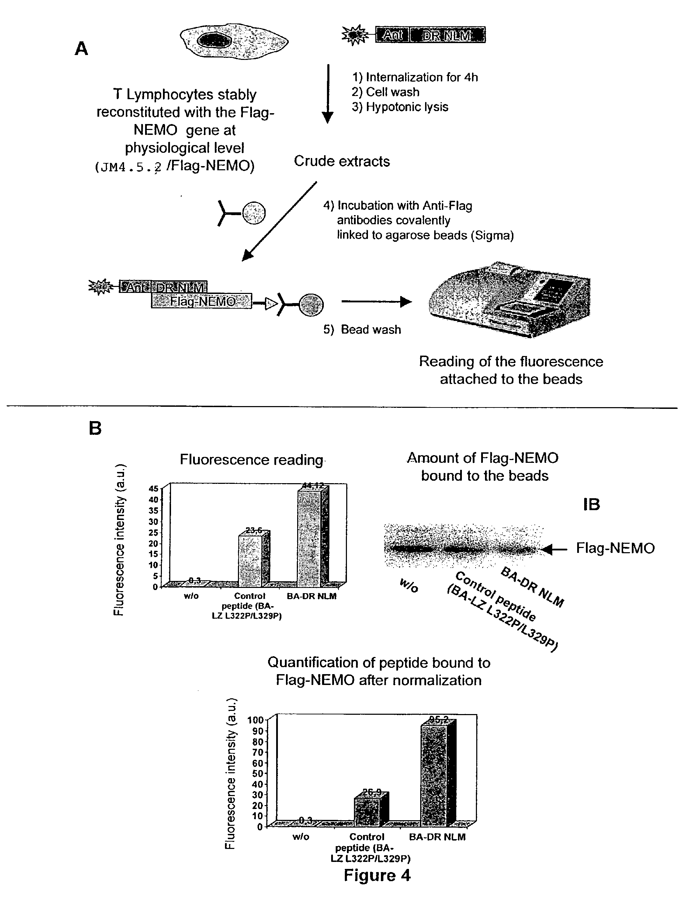

**Figure 4****: the peptide DR NLM interacts with the endogenous NEMO** A, overview of the method for identifying the protein target of DR NLM peptide. **B**, Fluorescence measurements of Flag-NEMO coated beads. T lymphocytes stably reconstituted with Flag-NEMO (JM4.5.2/Flag-NEMO) were treated in the absence (w/o) or in the presence of the indicated fluorescent peptides for 2h to allow peptide internalization. The control peptide (BA-LZ L322P/L329P) comprises the sequence of the wild type NLM. Following several cell washes to remove any peptide excess, the cells were lysed and the endogenous Flag-NEMO was pulled down using anti-Flag antibodies. The amount of peptide bound to NEMO was then examined by fluorescence (top left). To normalize all samples, the amount of NEMO coated to the beads was determined by Western blotting using anti-NEMO antibodies (top right) was used to normalize all samples (bottom).

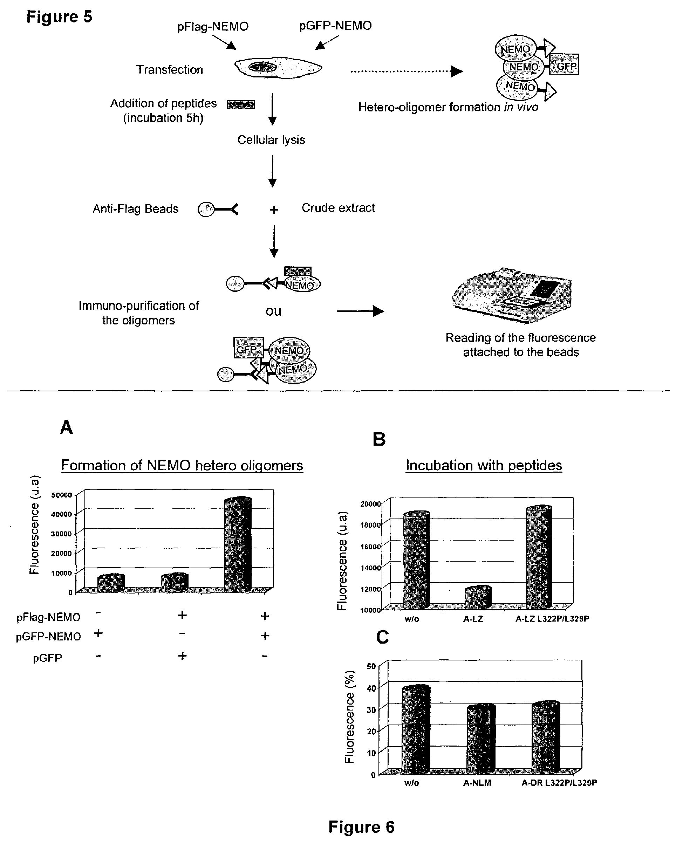

**Figure 5****: Schematic view of the cell based assay (cell-based assay) to probe the effects of different peptides on NEMO oligomerization**
**Figure 6****: DR NLM peptide does not inhibit NEMO oligomerization in cells A**, formation of hetero-oligomers with GFP-NEMO and FLAG-NEMO. **B**, **C**, Co-tranfected 293T cells with GFP-NEMO and Flag-NEMO were treated for 2 h with 20 µM of the peptides A-LZ, A-LZ L322P/L329P (B), A-NLM or A-DR NLM (C). The NEMO hetero oligomers were then immunopurified and the amount of the fluorescent NEMO subunit bound to hetero oligomers was examined by fluorescence as described in Example 1.I.

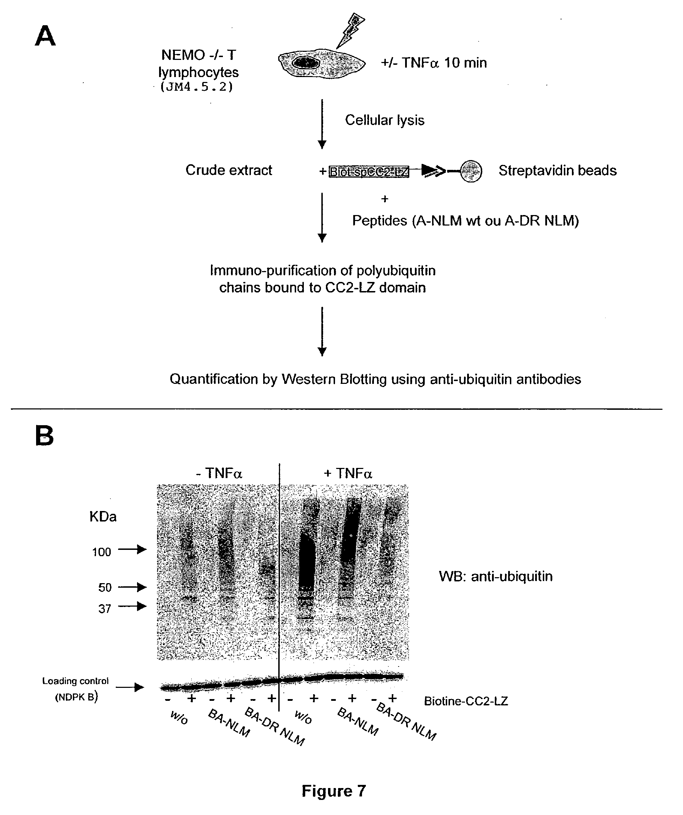

**Figure 7****: DR NLM impedes the interaction of cellular polyubiquitinylated proteins with the peptide CC2-LZ comprising the CC2 and LZ domains of NEMO. A**, overview of the method for probing the interaction of polyubiquitinylated proteins to the peptide spCC2-LZ. **B**, Western blotting of polyubiquitinylated proteins binding to the peptide spCC2-LZ with or without stimulation by TNF-α, and with (+) or without (-) the peptide BA-NLM or BA-DR NLM.

### EXAMPLES

### Example 1 - Materials and methods

### A.- Cell Culture, Biological Reagents and Antibodies

Absence of endotoxin contamination was checked in the RPMI 1640 medium (Invitrogen) and in the fetal calf serum (Biowest) used in cell culture. Jurkat (clone 20) cells were maintained in RPMI 1640 supplemented with 10% fetal calf serum. Stable cell lines (70Z/3-C3, C.N.C.M. I-3004) were obtained after electroporation of 70Z/3 cells in the presence of a cx12lacZ-κB plasmid containing three tandem copies of an NF-κB binding site in the interleukin-2 promoter upstream from the lacZ reporter gene as described in Agou *et al.,* 2004b. They were maintained in RPMI 1640 supplemented with 10% fetal calf serum and 50 µM β-mercaptoethanol (Sigma). Stable cell lines (JM4.5.2/Flag-NEMO) were obtained after electroporation of NEMO deficient T lymphocytes (JM4.5.2, Harhaj *et al.* 2000) in the presence of a pcDNA3 plasmid containing the sequence of murine Flag-NEMO (Flag is an hydrophilic 8 amino acid peptide of sequence SEQ ID NO: 23 provided by Sigma). These human Jurkat leukemic T-cells were maintained in RPMI 1640 supplemented with 10% fetal calf serum.

The human embryonal kidney cell line 293T (American Type Culture Collection; Manassas, VA) was grown in DMEM medium (Invitrogen) supplemented with 100 units/ml penicillin and streptomycin (Invitrogen) and 10% fetal calf serum. LPS from *Salmonella abortus,* phorbol 12-myristate 13-acetate (PMA) and ionomycin were purchased from Sigma and the recombinant mouse IL-1β was purchased from BD Pharmingen. The polyclonal rabbit anti-NEMO serum was a gift from R.Weil and the specific antibodies were purified by immunoaffinity. The anti-GFP polyclonal antibody was from Oncogene. The anti-Flag M2 monoclonal antibody as well as the anti-ubiquitin antibody were from Sigma.

### B.- Peptide Synthesis and Purification

Peptides are synthesized as previously described (Agou *et al.,* 2004b). Sequences of peptides were already reported in the International Patent W02005027959 and some of them are represented in the following Table.

**Table 1: Sequence of NEMO derived peptides**

| Name | Sequence⁽¹⁾ | SEQ ID NO: | Theoretical mass (Da) | Experimental mass (Da) | Constructions with NEMO Human sequences | SEQ ID NO: |
|---|---|---|---|---|---|---|
| BODIPY-Ant (BA) | B-**CRQIKIWFQNRRMKWKK** | 3 | 2805.19 | 2805.06 ± 0.52 | | |
| BA-LZ | B-CRQIKIWFQNRRMKWKKL KAQADIYKADFQAERHAREKL VEKKEYLQEQLEQLQREFNKL | 4 | 8064.2 | 8063.9 ± 0.48 | B-CRQIKIWFQNRRMKWKKLKAQAD IYKADFQAEQAREKLAEKKELLQEQLE QLQREYSKL | 5 |
| LZ | LKAQADIYKADFQAERHAREK LVEKKEYLQEQLEQLQREFNK L | 6 | 5318.08 | 5318.21 ± 0.5 | LKAQADIYKADFQAERQAREKLAEKK ELLQEQLEQLQREYSKL | 7 |
| CC2 | SKGMQLEDLRQQLQQAEEALV AKQELIDKLKEEAEQHKIV | 8 | 4155.75 | 4155.86 +/- 0.53 | SKGMQLEDLKQQLQQAEEALVAKQEV IDKLKEEAEQHKIV | 9 |
| NLM | LKAQADIYKADFQAERHAREK | 10 | | | LKAQADIYKADFQAERQAREK | 18 |
| DR NLM | LKAQADIYKA**R**FQAERHAREK | 11 | 2570.94 | 2570.78 +/- 0.21 | LKAQADIYKA**R**FQAERQAREK | 12 |
| BA-DR NLM | B-CRQIKIWFQNRRMKWKKL KAQADIYKARFQAERHAREK | 13 | 5317,08 | 5316.38 +/- 0.26 | B-CRQIKIWFQNRRMKWKKLKAQAD IYKARFQAERQAREK | 14 |
| BR₇-DR NLM | B-CRRRRRRRLKAQADIYKAR FQAERHAREK | 15 | 4181.65 | 4181.60 +/- 0.69 | B-CRRRRRRRLKAQADIYKARFQAER QAREK | 16 |
| A-Cterm-LZ | RQIKIWFQNRRMKWKKLVE KKEYLQEQLEQLQREFNKL | 20 | | | RQIKIWFQNRRMKWKKLAEKKELLQ EQLEQLQREYSKL | 21 |
| spCC2-LZ | LEDLRQQLQQAEEALVAKQEL IDKLKEEAEQHKIVMETVPVLK AQADIYKADFQAERHAREKLV EKKEYLQEQLEQLQREFNKL | 17 | | | LEDLKQQLQQAEEALVAKQEVIDKLK EEAEQHKIVMETVPVLKAQADIYKADF QAERQAREKLAEKKELLQEQLEQLQR EYSKL | 19 |
| BA-LZ L322P/L329P | B-CRQIKIWFQNRRMKWKKL KAQADIYKADFQAERHAREKL VEKKEYPQEQLEQPQREFNKL | 22 | | | | |

| | | | | | | |
|---|---|---|---|---|---|---|
| (1) In peptides coupled to Bodipy, the N-terminus contains a cysteine residue for convenience of specific peptide coupling with the maleimide group as described in the Patent Application WO2005/027959. The sequences of antennapedia and poly-arginine (R₇) fused to the NEMO sequence (plain text) are highlighted in bold characters. Residues that may be involved in coiled-coil sequence are underlined and those that were replaced in the CC2 and LZ mutants and in NLM-DR are underlined in bold characters. B=Bodipy (The C-terminal Bodipy modification has been omitted from the sequences appearing in the Sequence Listing). Within the scope of the present invention it is contemplated that Bodipy and/or the N-terminal cysteine may be removed and used as described herein. | | | | | | |

### C.- Conditions for FACS Analysis

The internalization of the peptides is analyzed by FACS as described in the International Application WO 2005/027959.

### D.- NF-κB Inhibition Assays

4.5 x 10⁵ 70Z/3-C3 cells in 450 µl of 10% FCS-RPMI (Invitrogen) were incubated with 0-20 µM peptide BA-DR NLM or BA-NLM. After 2 h at 37°C, 200 µl of cell samples were transferred in duplicate to a 96-well microtiter plate. One well was treated for 4 h with either 15 µg/ml LPS, 20 ng/ml IL1-β or 100 ng/ml PMA. A control well was left untreated. After 4 h at 37°C, cells were centrifuged at 400 x g for 5 min at room temperature, the pellets were washed three times with cold PBS (200 µl), and the cells were lysed in 100 µl of lysis buffer (25 mM tris-phosphate buffer at pH7.8 containing 8 mM MgCl₂, 1 mM dithioerythreitol, 1% Triton X-100, 15% glycerol and a protease inhibitor mixture (Roche Applied Science, ref. 1836145)). The lysate was centrifuged at 1000 x g for 20 min at 4°C, and supernatant was kept on ice before performing the β-galactosidase assay. β-galactosidase assays were performed on 50 µl of the supernatant and in an assay mix containing 4 µl of Galacton-star chemiluminescent substrate and 196 µl of reaction buffer (Clonetech). The activity was measured with a plate luminometer (Berthold).

### E.- Inhibition Assays of Other Signalisation Pathways

Jurkat T cells (clone 20) were transiently transfected by a DEAE-dextran method with a reporter plasmid containing the luciferase under the control of a promoter inducible by the ERK and p38 pathway (SRE-luc; Courtois et al., 1997, Mol. Cell. Biol, 17:1441-1449), the NF-AT pathway or the AP1 pathway (respectively NF-AT-luc and AP1-luc; Northrop et al., 1993, J.Biol.Cell., 268:2917-2923). Briefly, cells were washed and resuspended at 10⁷ cells/ml in TBS containing 0.5 mg/ml of DEAE-dextran (Pharmacia). 1 µg per 2.10⁶ cells of reporter plasmid was added. After 45 min at room temperature, the cells were diluted with 10 volumes of TBS, centrifuged, and resuspended at 10⁶ cells/ml in 10% FCS-RPMI. After 24 h, cells were incubated with or without 5 µM NEMO-derived peptides (BA-NLM or BA-DR NLM) for 2 h. They were stimulated or mock-stimulated with 100 ng/ml PMA and 1 µg/ml ionomycin for 5 h, and finally lysed in luciferase buffer (25 mM Tris-phosphate pH 7.8 containing 8 mM MgCl₂, 1 mM DTE, 1% Triton X100, 15% glycerol). Luciferase measurement was carried out in a Berthold Luminometer.

### F.- Fluorescence Anisotropy

Anisotropy measurements were performed with a QuantaMaster® fluorometer (PTI), equipped with polarizing filters for excitation and emission, and using a photomultiplier tube in the L-configuration. All experiments were carried out in a microcuvette (60 µl) at 22°C, with excitation and emission wavelengths at 495 and 513 nm. The band pass of the excitation and emission wavelengths was 2 and 4 nm, respectively. Steady-state fluorescence anisotropy, expressed as millianisotropy units was measured as described Agou *et al.,* 2004a. All measurements were carried out in a 20 mM isoionic buffer pH 8 (10 mM acetic acide, 10 mM MES, 20 mM Tris), containing 20 mM KCl. Each data point is the result of 20 recording taken over a 2-min period.

### G.- Circular Dichroism Spectroscopy

Far-UV circular dichroism (CD) measurements were performed with an Aviv 215 spectropolarimeter with samples diluted in 10 mM NaPO₄ at pH 7.

### H.- Identification of Proteins Bound to Bodipy-conjugated peptides

5.10⁶ JM4.5.2/Flag-NEMO cells were seeded in 6-well plates (3 ml/well) and incubated with 20 µM of Bodipy-conjugated peptides (BA-NLM or BA-DR NLM) for 4 h at 37°C. Cells were centrifuged at 400 x g for 5 min, resuspended in 200 µl of an hypotonic buffer (10 mM TrisHCl pH 7.9, 1,5 mM MgCl₂, 10 mM KCl), left on ice for 20 min and lysed by several passage through a 26G needle. Lysates were clarified at 15 000 x g for 20 min and 80 µg of total proteins were incubated with 40 µl of agarose anti-Flag M2 beads for 1 h at 4°C. The Flag-NEMO protein were pulled down with the agarose anti-Flag M2 beads by centrifugation for 1 min. Beads were resuspended in 100 µl of hypotonic buffer and the fluorescence of NEMO linked peptide was read on microplate reader from SAFAS. NEMO coated beads were analyzed by Western blotting using anti-Flag M2 monoclonal antibodies to normalize the sample using the UN-SCAN-IT software.

### I.- Inhibition of NEMO oligomerization in cells

293T cells were transiently co-transfected by calcium-phosphate-DNA precipitation method with 2 µg of a plasmid pcDNA3 (Invitrogen) containing the sequence of FLAG-NEMO (pcDNA3-Flag-NEMO of sequence SEQ ID NO: 24) and 0.7 µg of a plasmid pcDNA3 containing the sequence of GFP-NEMO (pcDNA3-GFP-NEMO of sequence SEQ ID NO: 26). Empty vector is added up to a total amount of DNA of 4 µg per 6 cm-dish. 24 h after transfection, cells were resuspended and incubated with or without 20 µM of peptides (A-NLM or A-DR NLM) for 7 h at 37°C. Cells were resuspended and washed twice with PBS to remove excess peptides and lysed in 150 µl of lysis buffer containing a protease inhibitor mixture as indicated above (Example l.D). Lysates were clarified at 15 000 x g at 4°C for 20 min. The amount of proteins in the clarified lysat is determined by the Bradford method with the kit DC Biorad. 100 µg of total proteins were diluted in 200 µl of interaction buffer (50 mM TrisHCl pH 7,5, 1% triton X100, 1 mM DTE, 300 mM NaCl) and incubated 1 h at 4°C with 40 µl of agarose anti-Flag M2 beads (Sigma). The oligomers Flag-NEMO/GFP-NEMO were pulled down with the anti-Flag M2 beads and washed twice with the interaction buffer. The fluorescence of GFP-NEMO were directly read on the beads with a flurorescence microplate reader SAFAS and the clarified lysates were analysed by Western blotting using anti-Flag, anti-GFP and anti-NDPK-B antibodies (Kraeft et al., 1996; Exp Cell Res;227:63-69) to normalize the results.

### J.- Identification of polyubiquitinylated proteins bound to the peptide spCC2-LZ comprising the CC2 and LZ domains of NEMO.

24.10⁷ JM4.5.2 cells were stimulated or not with 10 ng/ml of TNFα for 10 minutes at 37°C, washed twice with cold PBS and lysed in 900 µl of lysis buffer containing a protease inhibitor mixture as indicated in Exampel 1.D. Lysates were clarified at 15 000 x g at 4°C for 30 min. 150 µl of proteins were incubated 2 h at 4°C with 150 µl of streptavidin magnetic beads (MagPrep®, Novagen) pre-incubated 1 h at 4°C with 10 µg of biotynilated-CC2-LZ and with or without 20 µM of peptides (BA-NLM or BA-DR NLM). Beads were washed 4 times in buffer A (10 mM Hepes pH 7.5, 150 mM NaCl, 8 mM MgCl₂, 10 % glycérol, 0.1 mM DDM and 1 mM DTE) and resuspended in 30 µl of laemmli buffer containing 6 M Urea. Samples were analysed by Western blotting using anti-ubiquitin antibodies (Sigma) as well as anti-NDPK-B antibodies for loading controls.

### Example 2 - DR NLM peptide binds in vitro to the oligomerization domain of NEMO

Previous studies of circular dichroism and gel filtration show that the NLM and DR NLM peptides do not form oligomeric coiled-coil structures and behave as monomer at a peptide concentration up to 100 µM (data not shown). Then, unlike the LZ peptide, DR NLM peptide as well as NLM peptide are unable to self-associate. Self association of LZ peptide is described by Agou *et al.,* 2004b.

Here the inventors confirm that DR NLM inhibits the NF-kB pathway after activation of the pre-B 70Z3 lymphocytes with a phorbol ester (PMA), a pro-inflammatory cytokine (IL-1) and an endotoxin (LPS) (Figures 1A and 1B). The IC₅₀ measured for these various stimuli are similar (0.9 µM +/- 0.1). Moreover, the use of a variety of internalization peptides (Tat, Antennapedia, R7, R9) yields similar IC₅₀ values, suggesting that internalization is not the limiting step.

To analyse whether DR NLM binds the MOD of NEMO, fluorescent labelled BA-DR NLM (10 µM) was incubated with an increasing concentrations of spCC2-LZ and the complex formation was monitored by fluorescence polarization (P) and results are expressed as anisotropy (A) according to the formula A=2P/(3-P). The concentration of spCC2-LZ was determining by amino acid analysis (Vinolo et al; 2006). Data points were fitted to the binding isotherm equation which gives a dissociation constant of 17 µM. Inset, the same experiment was done with BR₇-DR NLM (10 µM) which gives a similar dissociation constant (K_{D} = 21 µM).

DR NLM form a stable MOD/peptide complex with an affinity of 17 µM and a stoichiometry of 3:1 (Fig. 2A). This suggests that DR NLM peptide interacts preferentially with the trimer of MOD. This interaction does not depend on the cell permeable sequence because DR NLM fused to its N-terminus with the antennapedia peptide (BA-DR NLM) or the poly R₇ peptide (BR₇-DR NLM) displays similar dissociation constants (Inset, Fig. 2A). The binding site is located by fluorescence polarization using various peptides that mimic several regions of MOD (Fig. 2B). This site corresponds to the C-terminal part of NEMO's LZ subdomain (residues 315-336). This site was previously determined to be essential for NEMO function through point mutation analysis (Agou *et al.,* 2004a).

These studies probing the selectivity of DR NLM-induced NF-κB inhibition in various cell types capture the most attractive properties of the peptide.

Results of the inhibition assays described in Example 1.E are depicted in Figure 3. They demonstrate that NLM-DR does not abrogate at least four other pathways including AP1, ERK, p38 and NF-AT in T-lymphocytes in responses to PMA and ionomycin, indicating that the DR NLM peptide, but not the NLM peptide, inhibits specifically the NF-κB pathway (Fig. 3).

### Example 3 - DR NLM directly interacts with NEMO in cells.

To address the question whether the inhibition observed by the DR NLM peptide was due to a direct interaction with NEMO, we used NEMO-deficient T lymphocytes (JM4.5.2 cell lines) stably reconstituted with the gene of Flag-NEMO protein (JM4.5.2/Flag-NEMO) as described in Example 1.A. After incubation of the cells with BA-NLM-DR fluorescent labelled peptide (20 µM) or the fluorescent labelled NLM control (BA-LZ L322P/L329P) for 4 h, the cells were lysed and the crude extracts were incubated with anti-Flag antibodies covalently linked to agarose beads as described in Example 1.H (see Fig. 4A for an overview of the method). Following the pull-down of the beads, the fluorescence attached to the beads was measured, (Example I.H), providing an assay of the amount of peptide bound to NEMO (Fig. 4A and 4B). This assay was normalized relative to the expression level of Flag-NEMO protein in each sample by Western blot using anti-Flag M2 monoclonal antibody. Figure 4B shows that DR NLM binds specifically to NEMO, showing a perfect correlation between the effect of a given peptide in inhibiting the activation of the pathway and its ability to interact with NEMO.

### Example 4 -DR NLM does not interfere with NEMO oligomerization.

In order to better analyze the molecular mechanism of the inhibition of the NF-κB pathway by DR NLM peptide, the Inventors have developed an assay to probe NEMO oligomerization in cells as described in Example 1.I (see Figure 5 for an overview of the cell-based assay). For this, two plasmids were used, expressing respectively GFP-NEMO and Flag-NEMO fusion proteins. The plasmids were co-transfected in human 293T cells to overexpress exogenous GFP-NEMO and Flag-NEMO fusion proteins and enforce NEMO oligomerization. The cells were then submitted to detergent lysis and Flag-NEMO proteins were immunopurified using anti-Flag agarose beads and Flag peptide as described in Example 1.I. The fluorescence associated with beads that results from an association of Flag-NEMO with GFP-NEMO subunits within hetero-oligomers allow an estimate of the amount of oligomers present in the cellular extracts. Controls including the expression of GFP-NEMO alone or the co-expression of Flag-NEMO and GFP proteins do not show any fluorescence associated with the anti-Flag beads (Fig. 6A).

The BA-LZ peptide inhibits *in vitro* NEMO oligomerization (Agou *et al.,* 2004b). Then, BA-LZ was used as positive control to validate the above fluorescence cell-based assay. Under the conditions of this assay, the fluorescence emission of Bodipy does not interfere with the measure of the fluorescence emission of GFP. As shown in Figure 6B, the incubation with BA-LZ peptide (20 µM) results in a strong decrease in the amount of fluorescent NEMO subunit, indicating that the BA-LZ peptide inhibits NEMO oligomerization in cells. In contrast, when a homologous peptide (BA-LZ L322P/L329P) bearing a double mutation within the coiled-coil interface is used, or when the cells are treated without peptide, a higher amount of the fluorescent NEMO subunit was recovered. Interestingly the formation of NEMO oligomers was equivalent in the absence or in the presence of the mutant peptide (BA-LZ L322P/L329P) indicating that no dissociation of NEMO oligomers was observed with this mutant peptide .

Using this cell based assay, the inventors have examined the capacity of the peptide BA-DR NLM to alter NEMO oligomerization. Figure 6C shows the same amount of NEMO oligomers in the absence or in the presence of BA-DR NLM or BA-NLM peptides (20 µM), despite the ability of BA-DR NLM to bind to the NEMO protein. In conclusion, in cells experiments point to a new mechanism of NEMO inhibition independent of its state of oligomerization.

### Example 5 - DR NLM inhibits in cells the interaction between CC2-LZ and polyubiquitinylated proteins.

Recent reports showed that NEMO also acts as a sensor of Lys 63-linked polyubiquitination (Ea *et al.,* 2006; Wu *et al.,* 2006). Thus, the Inventors examined whether DR NLM impedes the interaction between NEMO and the K63 polyubiquitin chains. To this end, they developed a pull down assay which uses the a peptide comprising the CC2 and the LZ domains of NEMO as a bait to pull down the polyubiquitinylated proteins as described in Example I.J and depicted in Figure 7A. The Biotinylated-CC2-LZ (Bio-CC2-LZ) coated on magnetic beads was co-incubated in the absence or in the presence of A-NLM or A-DR NLM peptides with crude extracts from NEMO deficient T lymphocytes (JM4.5.2 cell lines) stimulated or not with TNFα. After extensive washing of beads, all mono and polyubiquitinylated proteins bound to CC2-LZ were analysed by Western blot using anti-ubiquitin antibody (Sigma). As shown in Figure 7B, DR NLM peptide abolishes the CC2-LZ interaction with the cellular polyubiquitinylated proteins in a TNF-α dependent manner while no inhibition was observed in the absence or in the presence of the NLM peptide. These results show a significant loss of interaction between polyubiquitinylated proteins and CC2-LZ, indicating that DR NLM peptide inhibits the NF-κB pathway by blocking the interaction between NEMO and the polyubiquitinylated proteins. Correlatively, *in vitro* binding assays using fluorescence polarization show that the dissociation constant and the cooperativity index (Hill coefficient) of tag-free DR NLM for the Lys63-linked polyubiquitin chains (K_{D}= 250 µM, n = 1) are lower than those of tag-free NLM (K_{D} = 180 µM, n = 8). This indicates that the NLM peptide of NEMO represents a new ubiquitin binding motif and that the simple mutation D→R in the motif leads to a complete loss of interaction of NEMO with polyubiquitin chains.

The results outlined above and combined with those previously reported in the international Patent Application W02005027959 show that the region of NEMO comprising the CC2 and LZ domains acts as oligomerization domain as well as polyubiquitin chain binding domain. These dual properties of NEMO domain may allow to search for new compounds inhibiting the NF-κB signaling pathway by disrupting NEMO oligomerization, using a screening assay as described in Examples 1.11a), b) and c), or by disrupting the interaction between NEMO and the polyubiquitin chains, using a screening assay as described in Example 1.12.

### Example 6 - Screening assays based on CC2-LZ oligomerization

### A.- Search for compounds that compete for LZ peptide binding to the peptide CC2-LZ comprising the CC2 and LZ domains of NEMO

Anisotropy measurements are performed with a SAFAS Xenius fluorometer. All experiments are carried out in a black microplate Greiner (100 µl) at 20°C with excitation and emission wavelenghts at 495 and 515 nm. The band pass of the excitation and emission wavelengths is 8 nm. R₇-LZ Bodipy labeled (BR₇-LZ) and R₇-LZ peptides are indicated in Table 2 and the recombinant His-CC2-LZ protein is purified as described by Agou *et al.,* 2004b.

BR₇-LZ (5 µM) and R₇-LZ (20 µM) are incubated at 25°C with the target His-CC2-LZ (10 µM) to induce the formation of a stable complex in 100 µl of Tris/MES buffer (10 mM acide acétique, 10 mM MES) at pH 8 containing 50 mM KCl. The non-fluorescent peptide which shows the same affinity as the fluorescent peptide allows to increase the amount of peptides linked to the target. Under these experimental conditions, the addition of His-CC2-LZ leads to an anisotropy increase of 20% relative to the anisotropy signal of free R₇-NLM-LZ*BR₇-LZ peptide (77 mA) indicating the formation of a complex His-CC2-LZ:BR₇-LZ. The His-CC2-LZ/R₇-LZ/BR₇-LZ mixture is then seeded on 96-well plastic plates containing drug compound candidates. Hits are selected for their capacity to decrease fluorescence anisotropy, reflecting the peptide dissociation from His-tag CC2-LZ target.

### B.- Search for compounds that induce a conformational switch toward an inactive conformation of the peptide CC2-LZ comprising the CC2 and LZ domains of NEMO

To develop this screening assay, Fluorescence Resonance Energy Transfer method (FRET) is used. The CC2-LZ peptide is conjugated at its N-terminus with a fluorophore donor such as Cy5/5 (GE Healthcare) and at its C-terminus with a fluorophore acceptor like BHQ3 (Biosearch Technologies). The doubly conjugated CC2-LZ is brought into contact with a molecule to be tested and the fluorescence is measured. Hits that induce an increase of fluorescence emission will be selected.

### C.- Search for compounds that inhibits CC2-LZ oligomerization

A screening assay which relies on Homogeneous time resolved fluorescence (HTRF) will also be set up for a higher throughput. Europium-Cryptate (fluorophore donor) and XL665 (fluorophore acceptor) conjugated antibodies that respectively recognize the biotine and the (His)₆ tag are commercially available (Cis-Bio inc.) The purification of His-tag CC2-LZ peptide is described in Agou *et al.,* 2004b. The biotine tagged CC2-LZ peptide is chemically synthezised or is purified from a recombinant *E.coli* strain such as AviTag (Avidity, inc). The main benefit of this method is that it is affortable and it diminishes the risk of false positive

### Example 7 - Screening assays based on polyubiquitin binding properties of the CC2 and LZ domains

### A.- Primary screening assay

In a first step K63 linked polyubiquitin chains (K63Ub ₂₋₇, Boston Biochem, Inc.) is immobilized on Ni-NTA HisSord plates (96 well plates,Qiagen, Inc). The fluorescent labelled CC2-LZ (*e.g.* B-CC2-LZ) or the fluorescent NLM peptide (*e.g.* B-DR NLM) is added to form fluorescent protein complexes with the K63 polyubiquitin chains. Hits that compete for the CC2-LZ or the NLM binding to K63 polyubiquitin chains is considered as positive and is selected.

### B.- Secondary screening assay

In a second step, all positive hits that derived from primery screenings described above are submitted to a secondary screening to examine their effects on NF-κB inhibition using the cell-based assay which was described in the W02005027959 patent.

Abbreviations used: BA-peptide, peptide fused to its N-terminus with the cell permeable sequence antennapedia and conjugated with the bodipy fluorophor; A-peptide, Antennapedia tagged peptide; R7-peptide, peptide fused to its terminus with the cell permeable polycationic peptide RRRRRRR; Tat, cell permeable peptide containing the sequence YGRKKRRQRRR; R9, cell permeable cationic peptide containing nine arginine residues; spCC2-LZ, synthetic polypeptide subdomain CC2-LZ; DDM, dodecyl maltoside; MES, morpholinoethane sulfonic acid; DTE, dithioerythrol.

### References

- Agou, F., Ye, F., Goffinont, S., Courtois, G., Yamaoka, S., Israël, A. and Véron, M. (2002) NEMO trimerizes through its coiled-coil C-terminal domain. J. Biol. Chem., 20, 17464-17465.
- Agou, F., Traincard, F., Vinolo, E., Courtois, G., Yamaoka, S., Israel, A. and Veron, M. (2004a) The trimerization domain of NEMO is composed of the interacting C-terminal CC2 and LZ coiled-coil subdomains. J Biol Chem, 279, 27861-27869.
- Agou, F., Courtois, G., Chiaravalli, J., Baleux, F., Coic, Y.M., Traincard, F., Israel, A. and Veron, M. (2004b) Inhibition of NF-kappa B activation by peptides targeting NF-kappa B essential modulator (nemo) oligomerization. J Biol Chem, 279, 54248-54257.
- Carter RS, Geyer BC, Xie M, Acevedo-Suarez CA, Ballard DW. (2001) Persistent activation of NF-kappa B by the tax transforming protein involves chronic phosphorylation of IkappaB kinase subunits IKKbeta and IKKgamma. J Biol Chem. Jul 6;276(27):24445-8. Epub 2001 Apr 26.
- Chan DC and Kim PS. (1998) HIV entry and its inhibition.Cell. 1998 May 29;93(5):681-4. Review. No abstract available.
- Courtois G, Whiteside ST, Sibley CH, Israel A. (1996) J Biol Cell 268: 2917-2923.
- Mike Chang, Lianshan Zhang, James P. Tam, and Elaine Sanders-Bush (2000) Dissecting G Protein-coupled Receptor Signaling Pathways with Membrane-permeable Blocking Peptides. J. Biol. Chem., Mar 2000; 275: 7021 - 7029.
- Döffinger, R., Smahi, A., Bessia, C., Geissman, F., Feinberg, J., Durandy, A., Bodemer, C., Kenwrick, S., Dupuis-Girod, S., Blanche, S., Wood, P., Rabia, S.H., Headon, D.J., Overbeek, P.A., Le Deist, F., Holland, S., Belani, K., Kumararatne, D.S., Fisher, A., Shapiro, R., Conley, M.E., Reimund, E., Kalhoff, H., Abinun, M., Munnich, A., Israël, A., Courtois, G.and Casanova, J.-L. (2001) X-linked anhidrotic ectodermal dysplasia with immunodeficiency is caused by impaired NF-κB signaling. Nature Genet., 27, 277-285.
- Ea, C.K., Deng, L., Xia, Z.P., Pineda, G. and Chen, Z.J. (2006) Activation of IKK by TNFalpha requires site-specific ubiquitination of RIP1 and polyubiquitin binding by NEMO. Mol Cell, 22, 245-257.
- Eckert, D.M., and Kim, P.S. (2001) Mechanisms of viral membrane fusion and its inhibition. Ann. Rev. Biochem., 70, 777-810
- Erickson et al., Biophys J., 2003, 85, 599-611
- Ghosh, S., May, M.J. and Kopp, E.B. (1998) NF-κB and Rel proteins: evolutionarily conserved mediators of immune responses. Annu. Rev. Immunol., 16, 225-260.
- Harhaj, E.W., Good, L., Xiao, G., Uhlik, M., Cvijic, M.E., Rivera-Walsh, I. and Sun, S.C. (2000) Somatic mutagenesis studies ofNF-kappa B signaling in human T cells: evidence for an essential role of IKK gamma in NF-kappa B activation by T-cell costimulatory signals and HTLV-I Tax protein. Oncogene, 19, 1448-1456.
- Yinling HU, Veronique BAUD, Takefumi OGA, Keun IL KIM, Kazuhiko YOSHIDA and Michael KARIN (2001) IKK? controls formation of the epidermis independently of NF-κB Nature 410, 710-714
- Inohara, N., Koseki, T., Lin, J., del Peso, L., Lucas, P.C., Chen, F.F., Ogura, Y. and Nunez, G. (2000) An induced proximity model for NF-κB activation in the Nodl/RICK and RIP signaling pathways. J. Biol. Chem., 275, 27823-27831.
- Israel, A. (2000) The IKK complex: an integrator of all signals that activate NF-κB? Trends Cell. Biol., 10, 129-133.
- J. Kevin Judice, Jeffrey Y. K. Tom, Wei Huang, Terri Wrin, Joann Vennari, Christos J. Petropoulos, and Robert S. McDowell (1997) Inhibition of HIV type 1 infectivity by constrained a-helical peptides: Implications for the viral fusion mechanism PNAS 94: 13426-13430.
- Karawawa et al., Biochem J., 2004, 381(Pt1), 307-12
- Karin, M. (1999) The beginning of the end: IκB Kinase (IKK) and NF-?B activation. J. Biol. Chem., 274, 27339 - 27342.
- Kovalenko, A., Chable-Bessia, C., Cantarella, G., Israël, A., Wallach, D. and Courtois, G. (2003) The tumor suppressor CYLD negatively regulates NF-κB signalling by deubiquitination. Nature, 424, 801-805.
- Kraeft SK, Traincart F, Mesnildrey S, Bourdais J, Veron M, Chen LB (1996) Exp Cell Res, 227: 63-69.
- Yao-Zhong Lin, SongYi Yao, Ruth Ann Veach, Troy R. Torgerson, and Jacek Hawiger (1995) Inhibition of Nuclear Translocation of Transcription Factor NF-κB by a Synthetic Peptide Containing a Cell Membrane-permeable Motif and Nuclear Localization Sequence. J. Biol. Chem., Jun 1995; 270: 14255 - 14258.
- May, M.J. et al. (2000) Selective inhibition of NF-κB activation by a peptide that blocks the interaction of NEMO with the IκB kinase complex. Science, 289, 1550-1554
- May, M.J., Marienfield, R.B. and Ghosh, S. (2002) Characterization of the IκB-kinase NEMO binding domain. J. Biol. Chem., 277, 45992-46000.
- Northrop JP, Ullman KS, Crabtree GR (1993) J Biol Chem 268: 2917-2923.
- Pawson, T. and Nash, P. (2003) Assembly of cell regulatory systems through protein interaction domains. Science, 300, 445-452.
- Joel L. Pomerantz and David Baltimore. (2002) Two Pathways to NF-?B Molecular Cell 2002 10: 693-695
- Poulaki V., Mitsiades C.S., Joussen A.M., Lappas A., Kirchhof B., Mitsiades N. (2002) Constitutive nuclear factor-kappaB activity is crucial for human retinoblastoma cell viability. Am J Pathol., 161, 2229-2240.
- Poyet, J.-L., Srinivasula, S.M., Lin, J-H, Fernandes-Alnmeri, T., Yamaoka, S., Tsichlis, P.N. and Alnemri, E.S. (2000) Activation of yhe IκB kinases by RIP via IKKγ/NEMO-mediated oligomerization. J. Biol. Chem., 275, 37966-37977.
- Poyet, J.-L., Srinivasula, S.M. and Alnemri, E.S. (2001) vClap, a caspase-recruitment domain-containing protein of equine Herpesvirus-2, persistently activates the IκB kinases through oligomerization of IKKγ. J. Biol. Chem., 276, 3183-3187
- Santoro, G., Rossi, A. and Amici, C. (2003) NF-κB and virus infection: who controls whom. EMBO J., 22, 2552-2560.
- Uwe Senftleben, Yixue Cao, Gutian Xiao, Florian R. Greten, Gertraud Krähn, Giuseppina Bonizzi, Yi Chen, Yinling Hu, Abraham Fong, Shao-Cong Sun, and Michael Karin. (2001) Activation by IKK of a Second, Evolutionary Conserved, NF-κB Signaling Pathway. Science August 24; 293: 1495-1499.
- Shaner et al., Nat. Biotech., 2004, 22, 1567.
- Souroujon M.C. and Mochly-Rosen D. (1998) Peptide modulators of protein-protein interactions in intracellular signaling. Nat Biotechnol., 16, 919-924.
- Eric D. Tang, Naohiro Inohara, Cun-Yu Wang, Gabriel Nuñez, and Kun-Liang Guan. (2003) Roles for homotypic interactions and transautophosphorylation in B kinase (IKK) activation. J Biol. Chem. Vol. 278, 38566-38570
- Tegethoff, S. et al. (2003) Tetrameric oligomerization of IκB kinase γ is obligatory for IKK complex activity and NF-κB activation. Mol. Cell. Biol., 23, 2029-2041
- Trompouki, E., Hatzivassilou, E., Tsichritzis, T., Farmer, H., Ashworth, A. and Mosialos, G. (2003) CYLD is a deubiquitinating enzyme that negatively regulates NF-κB activation by TNFR family members. Nature, 424, 793-796
- Vinolo E, Sebban H, Chaffotte A, Israel A, Courtois G, Veron M, Agou F (2006) J Biol Chem 281:6334-48.
- Wu, C.J., Conze, D.B., Li, T., Srinivasula, S.M. and Ashwell, J.D. (2006) Sensing of Lys 63-linked polyubiquitination by NEMO is a key event in NF-kappaB activation. Nat Cell Biol, 8, 398-406.
- Ebrahim Zandi, David M. Rothwarf, Mireille Delhase, Makio Hayakawa, and Michael Karin. (1997) The I?B Kinase Complex (IKK) Contains Two Kinase Subunits, IKKa and IKKβ, Necessary for I?B Phosphorylation and NF-?B Activation. Cell 91: 243-252.
- Zonana, J., Elder, M.E., Schneider, L.C., Orlow, S.J., Moss, C., Golabi, M., Shapira, S.K., Farndon, P.A., Wara, D.W., Emmal,S.A. and Ferguson, B.M. (2000) A novel X-linked disorder of immune deficiency and hypohydrotic ectodermal dysplasia is allelic to incontinentia pigmenti and due to mutations in IKK-γ (NEMO). Am. J. Hum. Genet. 67, 1555-1562.

## Claims

1. Method for primary screening for a potential modulator of the NF-κB pathway activation, **characterized in that** it comprises the following steps:
(a) bringing into contact (i) a peptide P1 comprising the CC2 and LZ regions of NEMO, said peptide P1 being labelled or not, and (ii) a peptide P2, labelled or not, selected from the group consisting of a peptide comprising at least 10 amino acid residues of the LZ region of NEMO, a peptide comprising at least 10 amino acid residues of the NLM region of NEMO, a peptide comprising DR-NLM, a peptide comprising the CC2 and LZ regions of NEMO, a peptide comprising at least 10 amino acid residues of the CC2 region of NEMO, and a peptide comprising a K63-linked polyubiquitinylated chain; in the absence of the substance S to be tested;
(b) bringing into contact said peptide P1, labelled or not, and said peptide P2, labelled or not, in the presence of the substance S to be tested;
(c) detecting the complexes between P1 and P2 obtained in step (a) by measuring an appropriate signal;
(d) detecting the complexes between P1 and P2 obtained in step (b) by measuring an appropriate signal;
(e) comparing the signal measured in (c) and the signal measured in (d) and selecting the substance S for which the ratio of the signal measured in (c)/signal measured in (d) is different from 1.

2. Method according to claim 1, **characterized in that** P1 is selected from the group consisting of the peptides of sequences SEQ ID NO: 1, 2, 17 and 19.

3. Method according to claim 1 or claim 2, **characterized in that** P2 is selected from the group consisting of the peptides of sequences SEQ ID NO: 1, SEQ ID NO: 2, SEQ ID NO: 6 to 12 and SEQ ID NO: 17 to 19.

4. Method according to any one of claims 1 to 3, **characterized in that** said peptide P1 is immobilized on a solid support.

5. Method according to claim 4, **characterized in that** said peptide P1 is coupled to a biotine group and said solid support consists of a streptavidin beads.

6. Method according to any one of claims I to 5, **characterized in that** the signal in steps (c) and (d) is measured by Western blot, by fluorescent anisotropy, by Fluorescent Resonance Energy Transfer (FRET) or by Homogeneous Time Resolved Fluorescence (HTRF).

7. Method according to any one of claims 1 to 6, **characterized in that** said peptide P1 is unlabelled, and said peptide P2 is labelled with a fluorescent marker M1.

8. Method according to claim 7, **characterized in that** said marker M1 is selected from the group consisting of: green fluorescent protein (GFP), cyan fluorescent protein, yellow fluorescent protein and a fluorophore comprising a maleimide group.

9. Method according to claim 7 or to claim 8, **characterized in that** said peptide P2 labelled with said marker M1 is selected from the group consisting of the peptides of sequences SEQ ID NO: 4, 5 and 13 to 16.

10. Method according to any one of claims 1 to 6, **characterized in that** said peptide P1 is labelled with a marker M2 and said peptide P2 is labelled with a marker M3, M2 and M3 being a couple of a fluorophore donor and a fluorophore acceptor.

11. Method according to claim 10, **characterized in that** the marker M2 consists of a first tag and an antibody directed against said first tag and labelled with a fluorophore donor, and the marker M3 consists of a second tag and an antibody directed against said second tag and labelled with a fluorophore acceptor, said two tags being different.

12. Method according to claim 11, **characterized in that** said tag of the marker M2 is a poly His sequence, and said tag of the marker M3 is biotine.

13. Method according to any one of claims 1 to 12, **characterized in that** said potential modulator is a potential activator and **in that** step (e) comprises comparing the signal measured in (c) and the signal measured in (d) and selecting the substance S for which the ratio of the signal measured in (c)/signal measured in (d) is inferior to 1.

14. Method according to any one of claims 1 to 12, **characterized in that** said potential modulator is a potential inhibitor and **in that** step (e) comprises comparing the signal measured in (c) and the signal measured in (d) and selecting the substance S for which the ratio of the signal measured in (c)/signal measured in (d) is superior to 1.

15. Method for primary screening for a potential modulator of the NF-κB pathway activation, **characterized in that** it comprises the following steps:
(a) bringing into contact (i) a peptide P1 selected from the group consisting of: a peptide comprising at least 10 amino acid of the NLM region of NEMO, and a peptide comprising DR NLM, said peptide P1 being labelled or not, and (ii) a peptide P2 comprising a K63-linked polyubiquitine chain, said peptide P2 being labelled or not, in the absence of the substance S to be tested;
(b) bringing into contact said peptide P1, labelled or not, and said peptide P2, labelled or not, in the presence of the substance S to be tested;
(c) detecting the complexes between P1 and P2 obtained in step (a) by measuring an appropriate signal;
(d) detecting the complexes between P1 and P2 obtained in step (b) by measuring an appropriate signal;
(e) comparing the signal measured in (c) and the signal measured in (d) and selecting the substance for which the ratio of the signal measured in (c)/signal measured in (d) is different from 1.

16. Method according to claim 15, **characterized in that** said peptide P1 is selected from the group consisting of the peptides of sequences SEQ ID NO: 1, 2, 10 and 17 to 19.

17. Method according to any one of claims 1, 15 and 16, **characterized in that** said peptide P2, comprising a K63-linked polyubiquitinylated chain, is immobilised on a solid support.

18. Method according to any one of claims 15 to 17, **characterized in that** said potential modulator is a potential activator and **in that** step (e) comprises comparing the signal measured in (c) and the signal measured in (d) and selecting the substance S for which the ratio of the signal measured in (c)/signal measured in (d) is inferior to 1.

19. Method according to any one of claims 15 to 17, **characterized in that** said potential modulator is a potential inhibitor and **in that** step (e) comprises comparing the signal measured in (c) and the signal measured in (d) and selecting the substance S for which the ratio of the signal measured in (c)/signal measured in (d) is superior to 1.

20. Method for primary screening for a potential modulator of the NF-κB pathway activation, comprising the following steps:
(a) bringing into contact a peptide P1 comprising the CC2 and LZ regions of NEMO with the substance S to be tested, said peptide P1 being labelled with the markers M2 and M3, *i.e.* being coupled coupled to both a fluorophore donor and to a fluoropohore acceptor;
(b) measuring the fluorescence emission in the absence of the substance S to be tested;
(c) measuring the fluorescence emission in the presence of the substance S to be tested;
(d) comparing the signal measured in (b) and the signal measured in (c) and selecting the substance S for which the ratio signal measured in (b)/signal measured in (c) is different from 1.

21. Method according to claim 20, **characterized in that** said peptide P1 is selected from the peptides of sequences SEQ ID NO: 1, 2, 17 and 19.

22. Method according to claim 20 or claim 21, **characterized in that** said fluorophore donor is coupled to the N-terminus of the CC2 domain, and said fluorophore acceptor is coupled to the C-terminus of the LZ domain.

23. Method according to any one of claims 20 to 22, **characterized in that** said potential modulator is a potential activator and **in that** step (d) comprises comparing the signal measured in (b) and the signal measured in (c) and selecting the substance S for which the ratio of the signal measured in (b)/signal measured in (c) is inferior to 1.

24. Method according to any one of claims 20 to 22, **characterized in that** said potential modulator is a potential inhibitor and **in that** step (d) comprises comparing the signal measured in (b) and the signal measured in (c) and selecting the substance S for which the ratio of the signal measured in (b)/signal measured in (c) is superior to 1.

25. Method for primary screening for a potential modulator of NF-κB pathway activation, **characterized in that** it comprises the following steps:
a) providing a cell deficient in endogenous NEMO, which is transformed with one or more polynucleotides that express at least a peptide P1 and a peptide P2 as defined in any one of claims 1 to 3;
b) applying the substance S to the cell;
c) detecting the formation of complexes between P1 and P2 in the absence of the substance S by measuring an appropriate signal;
d) detecting the formation of complexes between P1 and P2 in the presence of the substance S by measuring an appropriate signal;
e) comparing the signal measured in (c) and the signal measured in (d) and by selecting the substance S for which the ratio of the signal measured in (c)/signal measured in (d) is different from 1.

26. Method according to claim 25, **characterized in that** the peptide P1 is a fusion protein GFP-NEMO and the peptide P2 is a fusion protein Flag-NEMO, Flag being a hydrophilic 8 amino acid residues peptide.

27. Method according to claim 26, **characterized in that** steps (c) and (d) comprise:
c1) (or d1)) the cell lysis,
c2) (or d2)) the immunoprecipitation of Flag-NEMO, bound or not to GFP-NEMO, using antibody directed against the Flag moiety of Flag-NEMO, and
c3) (or d3)) the detection the fluorescence emission of GFP moiety of GFP-NEMO bound to the immunoprecipitated Flag-NEMO.

28. Method according to any one of claims 25 to 27, **characterized in that** said potential modulator is a potential activator and **in that** step (e) comprises comparing the signal measured in (c) and the signal measured in (d) and selecting the substance S for which the ratio of the signal measured in (c)/signal measured in (d) is inferior to 1.

29. Method according to any one of claims 25 to 27, **characterized in that** said potential modulator is a potential inhibitor and **in that** step (e) comprises comparing the signal measured in (c) and the signal measured in (d) and selecting the substance S for which the ratio of the signal measured in (c)/signal measured in (d) is superior to 1.

30. Method for secondary screening for an inhibitor of NF-κB pathway activation, comprising:
a) selecting a substance S potentially able to inhibit NF-κB pathway activation by a method according to any one of claims 14, 19, 24 and 29,
b) applying an activator of NF-κB pathway in the absence or in the presence of the substance S selected in a), to a cell comprising a reporter gene under the control of a promoter inducible by NF-κB;
c) detecting the expression of the reporter gene by measuring an appropriate signal in the absence of the substance S;
d) detecting the expression of the reporter gene by measuring an appropriate signal in the presence of the substance S; and
e) comparing the signal measured in (c) and the signal measured in (d) and selecting the substance for which the ratio of the signal measured in (e)/signal measured in (d) is superior to 1.

31. Method for secondary screening for an activator of NF-κB pathway activation, comprising:
a) selecting a substance S potentially able to activate NF-κB pathway activation by a method according to any one of claims 13, 18, 23 and 28,
b) providing a cell comprising a reporter gene under the control of a promoter inducible by NF-κB;
c) detecting the expression of the reporter gene by measuring an appropriate signal in the absence of the substance S;
d) detecting the expression of the reporter gene by measuring an appropriate signal in the presence of the substance S; and
e) comparing the signal measured in (c) and the signal measured in (d) and selecting the substance for which the ratio of the signal measured in (c)/signal measured in (d) is inferior to 1.

32. Method according to claim 30, **characterized in that** said activator of NF-κB is selected from the group consisting of: TNF-α, IL-1β, PMA, ionomycin, and LPS from *Salmonella abortus.*

33. Method according to any one of claims 30 to 32, **characterized in that** said reporter gene is the gene *lacZ* encoding the β galactosidase or the gene *luc* encoding the luciferase.

34. Method according to any one of claims 30 to 33, **characterized in that** said cell is a 70Z/3-C3 cell, deposited in the Collection Nationale de Cultures de Microorganismes (C.N.C.M.) on April 1, 2003, under the number I-3004.
